(19) Europäisches Patentamt / European Patent Office / Office européen des brevets

(11) **EP 2 506 499 B1**

(12) **FASCICULE DE BREVET EUROPEEN**

(45) Date de publication et mention
de la délivrance du brevet:
**21.05.2014 Bulletin 2014/21**

(51) Int Cl.:
*H04L 12/26* (2006.01)     *A61B 5/00* (2006.01)
*H04W 72/12* (2009.01)

(21) Numéro de dépôt: **12162602.2**

(22) Date de dépôt: **30.03.2012**

(54) **Procédé d'évaluation de la qualité des liens radio pour un réseau corporel sans-fil, procédé de transmission de messages pour un réseau corporel sans-fil et dispositifs pour la mise en oeuvre des procédés**

Verfahren zur Bewertung der Qualität von Funkverbindungen für ein KörperNetzwerk, Funkübertragungsverfahren für ein Nachrichtentext in einem KörperNetzwerk und drahtlose Geräte für die Durchführung der Verfahren

A method of evaluating the quality of radio links for a wireless body network, message transmission method for a wireless body network and devices for the implementation of the method.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.04.2011 FR 1152843**

(43) Date de publication de la demande:
**03.10.2012 Bulletin 2012/40**

(73) Titulaire: **Commissariat à l'Énergie Atomique et aux Énergies Alternatives**
**75015 Paris (FR)**

(72) Inventeurs:
• **Ben Hamida, Elyes**
  **38000 Grenoble (FR)**
• **Denis, Benoit**
  **38000 Grenoble (FR)**

(74) Mandataire: **Croonenbroek, Thomas Jakob et al**
**Innovincia**
**310 avenue Berthelot**
**69008 Lyon (FR)**

(56) Documents cités:
**WO-A1-2010/073180     US-A1- 2008 013 458**

• **REUSENS E ET AL: "Characterization of On-Body Communication Channel and Energy Efficient Topology Design for Wireless Body Area Networks", IEEE TRANSACTIONS ON INFORMATION TECHNOLOGY IN BIOMEDICINE, IEEE SERVICE CENTER, LOS ALAMITOS, CA, US, vol. 13, no. 6, 1 novembre 2009 (2009-11-01), pages 933-945, XP011345618, ISSN: 1089-7771, DOI: 10.1109/TITB.2009.2033054**
• **KIM ETRI D KASPAR SIMULA RESEARCH LABORATORY C GOMEZ TECH UNIV OF CATALONIA/I2CAT C BORMANN UNIVERSITAET BREMEN TZI E: "Problem Statement and Requirements for 6LoWPAN Routing; draft-ietf-6lowpan-routing-requirements-05 .txt", PROBLEM STATEMENT AND REQUIREMENTS FOR 6LOWPAN ROUTING; DRAFT-IETF-6LOWPAN-ROUTING-REQUIREMENTS-05 .TXT, INTERNET ENGINEERING TASK FORCE, IETF; STANDARDWORKINGDRAFT, INTERNET SOCIETY (ISOC) 4, RUE DES FALAISES CH- 1205 GENEVA, SWITZERLAND, no. 5, 22 février 2010 (2010-02-22), pages 1-32, XP015066785,**

## Description

<u>DOMAINE TECHNIQUE</u>

La présente invention concerne un procédé d'évaluation de la qualité des liens radio pour un réseau corporel sans-fil, un procédé de transmission de messages pour un réseau corporel sans-fil et des dispositifs pour la mise en oeuvre des procédés.

La présente invention concerne toute application nécessitant un échange de données entre des dispositifs sans fil portés à l'échelle d'un corps dont au moins certaines parties sont mobiles, par exemple un humain, mais aussi une machine en mouvement.

L'avènement à moyen terme de réseaux corporels sans-fil intelligents, autonomes, et capables de répondre aux besoins d'applications émergentes, dans des domaines aussi variés que la sécurité, la santé, le sport et le divertissement de masse, apparaît aujourd'hui comme inéluctable.

Dans ce contexte, le développement de protocoles de communication plus robustes et adaptés aux propriétés et contraintes inhérentes à ces réseaux corporels sans fil, essentiellement liées à mobilité ainsi que des phénomènes bien particuliers de propagation autour du corps humain (e.g. obstruction ou masquage des liens radio, etc.), constitue un enjeu stratégique important.

On cite à titre d'exemple des applications mettant en oeuvre des communications entre les différents dispositifs sans fil formant le réseau corporel :

- Les applications de navigation et de positionnement de groupes de personnes mobiles en environnements intérieurs et les services géo-localisés.
- La capture de mouvements, par exemple, pour le suivi du geste sportif ou pour des applications de divertissement et de jeux.
- La détection de posture, par exemple, pour la rééducation, le suivi de personnes vulnérables ou âgées, et la surveillance de personnes évoluant dans un environnement sinistré (e.g., pompiers à l'intérieur d'un bâtiment en feu, *etc.).*
- Les applications médicales telles que les implants cardiaques, pompes à insuline, surveillance/monitoring des signes vitaux (température, battements du coeur [ECG- electrocardiogramme] *etc.).*
- Les applications de type « *terminal éclaté* » (écrans, claviers, oreillettes non co-localisées).

<u>ART ANTERIEUR</u>

Avec les récentes avancées technologiques en matière d'intégration et de miniaturisation, et grâce au développement de technologies de communication sans fil bas débit et très basse consommation, e.g. Bluetooth™ Low Energy (BLE), Zigbee™ ou IR-UWB (pour Impulse Radio Ultra WideBand en langue anglaise), un nouveau champ applicatif a émergé sous le nom de réseaux corporels sans fils ou BAN (pour « Body Area Networks » en langue anglaise).

On connaît par exemple du document WO 2010/018517 un réseau corporel sans fil BAN (pour Body Area Network), qui est conçu pour créer un réseau de capteurs portés par exemple par un patient ou implantés dans celui-ci pour surveiller certains paramètres vitaux du patient.

Dans ce type d'application, des dispositifs sans-fil ont vocation à former un réseau sur ou à grande proximité du corps humain : on peut citer des exemples applicatifs emblématiques tels que le « terminal éclaté » (écrans, claviers, oreillettes non co-localisés), les équipements sportifs (cardio-fréquencemètre, montre, podomètre sur la chaussure) ou les équipements médicaux (monitoring cardiaque, cérébrale, musculaire nomades).

Un réseau corporel sans-fil (BAN) est généralement constitué par un ensemble de dispositifs sans-fil, contraints en termes d'énergie, de capacités de calcul et de stockage, attachés (ou implantés) sur (ou dans) le corps humain avec pour vocation la formation d'un réseau auto-organisé et autonome. Chaque dispositif sans fil est capable de collecter des informations locales, au moyen de capteurs embarqués (positions, température, ECG, etc.), et de communiquer avec les autres dispositifs en vue d'acheminer les données collectées vers un dispositif central, appelé également coordinateur. Ce coordinateur joue principalement le rôle de passerelle entre le réseau corporel sans fil et un réseau extérieur (par exemple l'internet, un réseau cellulaire, un autre réseau de capteurs, etc.).

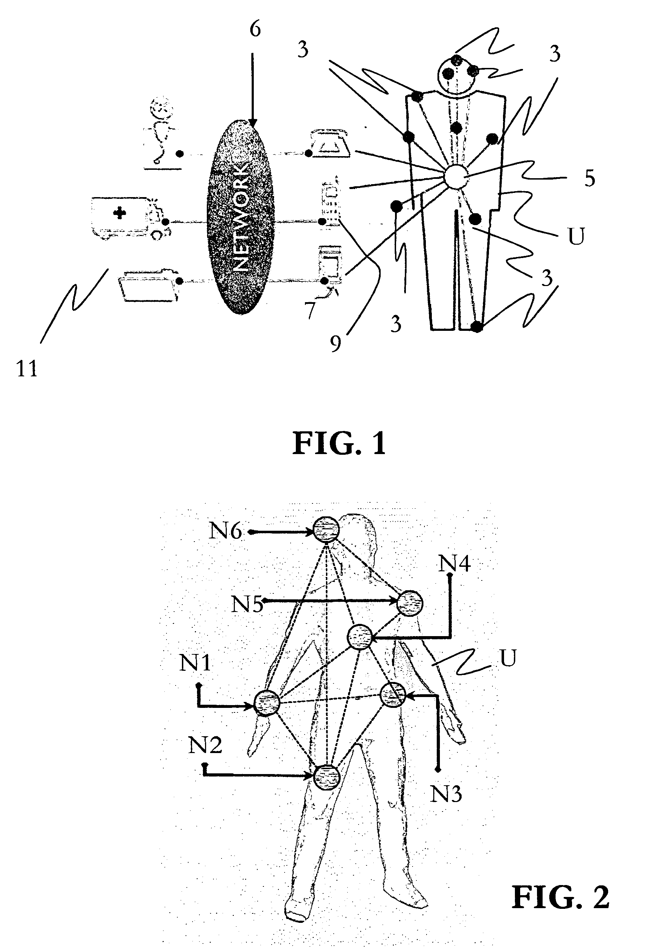

La figure 1 illustre un exemple de réseau corporel sans fil pour la surveillance médicale d'un patient. Sur cette figure, un patient ou utilisateur U porte sur lui divers dispositifs sans fil 3 équipés d'un ou de plusieurs capteurs, par exemple pour surveiller un implant, la pression du sang, le battement du coeur, la vision, la tenue du patient, le taux de sucre dans le sang etc.

Ces dispositifs sans-fil 3 sont reliés via un lien radio à un noeud central 5 (assumant en général le rôle de coordinateur du réseau sans fil) qui est configurée pour communiquer avec un réseau externe 6 par exemple via un point d'accès à un réseau WLAN 7, un téléphone cellulaire 9 etc. A ce réseau sont par exemple aussi connectés des services médicaux d'urgence et ou de surveillance 11, par exemple pour alerter une ambulance en cas de détection d'une anomalie par un des capteurs.

De par les caractéristiques inhérentes aux réseaux corporels sans fil, certaines techniques de communication et protocoles actuels, notamment ceux proposés dans le contexte des réseaux de capteurs (ou WSN pour « *Wireless Sensor Networks* » en langue anglaise), ou des réseaux personnels sans fil (ou WPANs pour « *Wireless Personal Area Networks* » en langue anglaise), ne sont pas adaptés.

En effet, les réseaux corporels sans fil introduisent de nombreuses nouvelles contraintes, liées essentiellement aux capacités limitées des dispositifs sans fil, à la nature du canal de propagation, à la mobilité du corps humain, ainsi qu'à la topologie particulière du réseau, nécessitant la mise en oeuvre de nouvelles stratégies de communication plus adaptées.

Un exemple très simple permet d'illustrer plus en détail ces propos. On considère que chacun des dispositifs sans-fil 3 est un des noeuds d'un réseau corporel BAN, qu'il produit par sa fonction ses propres informations pour être transmises sur ce réseau, et qu'il peut aussi servir de relais pour d'autres capteurs du réseau pour l'acheminement des données.

Pour sauvegarder l'énergie et ainsi augmenter l'autonomie du réseau, il peut être préférable de transmettre par exemple des données venant d'un podomètre fixé à la cheville via un dispositif sans-fil servant de relais implanté au niveau de la hanche vers le noeud central coordinatrice 5 au lieu de transmettre ces données directement sans relais à ce noeud central 5. Dans ce contexte, on doit également prendre en compte que la puissance d'émission est réduite, pour répondre aux standards IEEE 802 fixant les puissances d'émission admissibles pour des réseaux personnels.

Toutefois, la mobilité de l'entité portant ces dispositifs sans-fil (e.g. le patient, l'humain) impose d'autres restrictions.

Si on considère par exemple d'une part un capteur porté au niveau du poignet d'un humain et d'autre part que cet humain marche d'un pas vif, le bras se balance lors du mouvement et se trouve régulièrement au niveau ou dans son dos. De la sorte, si le noeud central 5 (ou le coordinateur) est portée au niveau du buste à l'avant, les données peuvent ne pas être transmises directement à ce noeud central 5 étant donné que lorsque le bras se trouve en position arrière, aucun lien radio ne peut être établi de façon suffisamment fiable avec le noeud central 5 dû à un masquage des ondes radio et / ou la faible puissance de transmission des dispositifs sans-fil.

Pour pallier ces difficultés, il est donc préférable de prévoir un noeud ou dispositif sans-fil servant de relais qui peut toujours établir une liaison fiable d'une part avec le dispositif sans-fil porté au poignet et d'autre part, soit avec un autre dispositif sans-fil, soit directement avec le noeud central 5.

Un article de A. Becher et Al. « Towards Short-Term Wireless Link Quality Estimation. In Hot Emnets, 2008 », concerne des réseaux de capteurs sans fil et introduit la notion de lien fiable à court terme et leur utilisation potentiel dans le routage lors de l'acheminement des données.

Bien que cet article traite des liens radio de faible puissance et suggère l'exploitation de liens radio possédant une fiabilité à court terme, il ne tient pas compte de la spécificité des réseaux corporels et en particulier de la mobilité entre un émetteur et un récepteur.

La présente invention vise à palier les inconvénients précités, au moins en partie en proposant un procédé d'évaluation de la qualité des liens radio pour un réseau corporel sans-fils qui permette déjà d'identifier et de qualifier une autre catégorie de liens potentiellement intéressants pour le routage et le relais d'information (de paquets) à travers le réseau corporel et par la suite, à chaque instant une meilleure exploitation de tous les liens radio disponibles du réseau corporel.

Plus précisément, la présente invention vise à améliorer au moins partiellement et en fonction du contexte applicatif les performances des réseaux corporels par exemple en termes de connectivité, consommation d'énergie, latence, fiabilité, taux de livraison des données, fiabilité des communications, robustesse des protocoles face à la variation dynamique des conditions de propagation et/ou à la mobilité du corps humain.

A cet effet, la présente invention propose un procédé d'évaluation de la qualité des liens radio pour un réseau corporel sans-fils comprenant au moins un premier et un second dispositifs sans-fil formant un réseau corporel et susceptibles de communiquer entre eux, au moins l'un des deux dispositifs sans fil est susceptible d'être mobile par rapport à l'autre dans lequel

- on exploite des messages reçus par au moins un des dispositifs sans-fil et on mesure la qualité instantanée des liens radios correspondant et on estime des durées pendant et entre lesquelles un lien radio est fiable,
  **caractérisé en ce que**
- on calcule un indicateur de fiabilité des durées estimées et
  on classifie les liens radio en fonction de l'indicateur de fiabilité en au moins deux catégories dont une catégorie relative aux liens radio fiables par intermittence tenant compte de la mobilité des dispositifs sans fil du réseau corporel.

Ainsi, on introduit une nouvelle catégorie de liens relative aux liens radio fiables par intermittence qui tient compte de la mobilité des dispositifs sans fils les uns par rapport aux autres.

Grâce à l'invention, on peut donc identifier la présence de liens fiables sur le court terme (ou liens dont la fiabilité est intermittente) et en estimer les principales caractéristiques, notamment la durée de contact (durée de temps pendant laquelle un lien radio peut être établi et est fiable) et la durée d'inter-contact (durée de temps nécessaire avant que le lien ne se rétablisse après une coupure de la connectivité).

Cela permet ensuite de prédire l'apparition/disparition de ces liens radio, ainsi que les durées de contact/inter-contact respectives, en vue d'améliorer les performances des protocoles de communication (par exemple le routage et le relais des paquets, ordonnancement des communications, etc.), en termes de consommation d'énergie, de latence, de taux de livraison des données, *etc.*

En effet, en reprenant l'exemple du dispositif sans-fil fixé au poignet, un lien radio direct entre ce dispositif sans-fil et le noeud central peut être établi de façon intermittente, ce qui permet de réduire davantage la puissance d'émissions radio et donc in fine la consommation d'énergie du réseau corporel.

Selon une ou plusieurs caractéristiques du procédé, prises seules ou en combinaison :

- les messages échangés entre les dispositifs sans-fils sont des messages de service, des messages de données ou une combinaison de ces messages,
- on échange ces messages de façon périodique ou pseudopériodique,
- on échange ces messages selon une fréquence variable,
- on évalue la qualité instantanée des liens radio en mesurant au moins un indicateur radio tel que RSSI, LQI, ou SNR,
- on évalue la qualité instantanée des liens radio en mesurant un paramètre de connectivité,
- on détermine le paramètre de connectivité et / ou l'indicateur radio de façon binaire ayant une valeur « 1 » ou une valeur « 0 »

$$L_{i,j}^{[t]} = \{0,1\} \text{ pour tout instant } t, \text{ entre un noeud } i \text{ et } j, i \neq j.$$

- par exemple

$$L_{i,j}^{[t]} = \begin{cases} 1 & \text{si le lien est actif par exemple si un échange de message a abouti} \\ 0 & \text{si le lien est inactif par exemple si un échange de message a échoué.} \end{cases}$$

- en variante

$$L_{i,j}^{[t]} = \begin{cases} 1 & \text{si l'indicateur radio est supérieur ou égal à un seuil} \\ 0 & \text{si l'indicateur radio est inférieur à un seuil} \end{cases}$$

- pour classifier les liens radio selon au moins deux catégories, on détermine à partir du paramètre de connectivité ou à partir de l'indicateur radio pour respectivement deux dispositifs sans-fil, des durées de contact et d'inter-contact et on détermine la présence de motifs répétitifs de contact par intermittence,

- la durée de contact, $\hat{C}_{i,j}^{[t]}$, est calculée comme étant la durée d'une séquence binaire

$$L_{N+M} = \{L_{i,j}^{[1]}, L_{i,j}^{[2]}, ..., L_{i,j}^{[N+M]}\} \in \{1\}^N \text{ et } \{0\}^M \text{ et telle que } \frac{N-M}{N} \geq \gamma_F, \text{ où } \gamma_F \text{ est un seuil de fiabilité du lien radio,}$$

- la durée d'inter-contact, $\hat{I}_{i,j}^{[t]}$, est calculée comme étant la durée d'une séquence binaire

$$L_{N+M} = \{L_{i,j}^{[1]}, L_{i,j}^{[2]}, ..., L_{i,j}^{[N+M]}\} \in \{1\}^N \text{ et } \{0\}^M \text{ et telle que } N \leq \gamma_{UP} \text{ où } \gamma_{UP} \text{ est le nombre maximal de « 1 » toléré durant la période d'inter-contact,}$$

- on calcule les durées finales de contact et d'inter-contact selon les formules suivantes :

$$C_{i,j}^{[t]}(\alpha_{CT}) = \alpha_{CT} \times C_{i,j}^{[t-1]} + (1 - \alpha_{CT}) \times \hat{C}_{i,j}^{[t]}$$

$$I_{i,j}^{[t]}(\alpha_{CT}) = \alpha_{CT} \times I_{i,j}^{[t-1]} + (1 - \alpha_{CT}) \times \hat{I}_{i,j}^{[t]}$$

où

- $\hat{C}_{i,j}^{[t]}$, respectivement $\hat{I}_{i,j}^{[t]}$ est l'estimation instantanée de la durée de contact, respectivement inter-contact ;

- $C_{i,j}^{[t]}$, respectivement $I_{i,j}^{[t]}$ est l'estimation finale à l'instant *t* de la durée de contact respectivement inter-contact; et

- $\alpha_{CT}$ est un facteur d'oubli.

- on calcule un coefficient de variation glissant, $V_{i,j}^{[t]}$, sur une fenêtre de taille $W_V$ qui est défini comme étant le rapport entre l'écart type et la moyenne des estimations sur une fenêtre glissante de taille $W_V$ et on le compare à un seuil $\gamma_V$ de sorte si le coefficient de variation glissant , $V_{i,j}^{[t]}$ est inférieur au seuil $\gamma_V$, un lien est classifié comme fiable par intermittence et s'il est supérieur ou égal au seuil, sinon il est classifié comme étant non fiable.
- on mémorise pour chaque dispositif sans-fil la fiabilité des liens radio dans une table de voisinage.

L'invention concerne également un dispositif d'évaluation de la qualité des liens radio pour un réseau corporel sans-fils comprenant plusieurs dispositifs sans-fil comprenant au moins un premier et un second dispositifs sans-fil formant un réseau corporel et susceptibles de communiquer entre eux, au moins l'un des deux dispositifs sans fil est susceptible d'être mobile par rapport à l'autre , pour la mise en oeuvre d'un procédé tel que défini ci-dessus, caractérisé en ce qu'il comprend des moyens configurés pour

- exploiter des messages reçus par au moins un des dispositifs sans-fil et mesurer la qualité instantanée des liens radios correspondant
- estimer des durées pendant et entre lesquelles un lien radio est fiable,
- calculer un indicateur de fiabilité des durées estimées et
- classifier les liens radio en fonction de l'indicateur de fiabilité en au moins deux catégories dont une catégorie relative aux liens radio fiables par intermittence tenant compte de la mobilité des dispositifs sans fil du réseau corporel.

L'invention concerne également un procédé de transmission de messages à travers un réseau corporel sans-fil caractérisé en que l'on évalue la qualité des liens radio pour un réseau corporel sans-fils selon un procédé tel que défini ci-dessus pour le routage ou le relayage et en ce que pour la transmission des messages, on achemine les messages via un lien radio fiable par intermittence. Ce procédé peut comprendre en outre une ou plusieurs des caractéristiques suivantes, prises seules ou en combinaison :

- on achemine les messages pendant une période pour laquelle le lien radio fiable par intermittence est considéré comme fiable,
- on favorise la transmission d'un message via un lien radio fiable par intermittence pour optimiser au moins un des paramètres du groupe suivant : consommation d'énergie, latence, taux de livraison des données,
- on ordonnance des mesures de distance entre les dispositifs sans-fil en tenant compte de la prédiction d'apparition et/ou de disparition des liens fiables par intermittence.

L'invention a également pour objet un dispositif de transmission de messages à travers un réseau corporel sans-fil pour la mise en oeuvre d'un procédé de transmission de messages tel que défini ci-dessus caractérisé en qu'il comprend des moyens configurés pour évaluer la qualité des liens radio pour un réseau corporel sans-fils pour le routage d'échange de données et /ou relayage de paquets entre les dispositifs sans-fil selon un procédé d'évaluation de la qualité des liens radio pour un réseau corporel sans-fils tel que défini ci-dessus et des moyens configurés pour acheminer les messages via un lien radio fiable par intermittence pendant une période pendant laquelle le lien radio fiable par intermittence est considéré comme fiable.

D'autres avantages et caractéristiques apparaîtront à la lecture de la description de l'invention, ainsi que des figures suivantes sur lesquelles :

- la figure 1 est un schéma illustrant un réseau corporel sans fil,
- la figure 2 est un schéma d'un réseau corporel sans fil selon l'invention,
- la figure 3 montre trois graphiques en fonction du temps pour respectivement trois liens radio différents,
- la figure 4 est un organigramme montrant différentes étapes du procédé selon l'invention,
- la figure 5 montre six graphiques en fonction du temps montrant d'une part l'atténuation de la puissance reçue et d'autre part la connectivité (i.e. la réception possible de paquets) pour trois liens radio différents,
- la figure 6 est un graphique montrant l'interprétation de la connectivité d'un lien radio en termes de durées de contact et d'intercontact en fonction du temps selon une approche déterministe,
- la figure 7 est un graphique montrant l'interprétation de la connectivité d'un lien radio en termes de durées de contact et d'intercontact en fonction du temps selon une approche probabiliste,
- la figure 8 est un schéma synoptique d'un dispositif selon l'invention pour la mise en oeuvre du procédé de la figure 4,
- la figure 9 est un schéma d'un réseau corporel sans fil selon l'invention présentant certains liens actifs pour illustrer le procédé selon l'invention,
- la figure 10 montre trois graphiques concernant l'évaluation de la qualité des liens,
- la figure 11 montre un graphique d'une architecture de communication de réseau corporel,
- la figure 12 montre un graphique comparatif du taux de livraison de paquets pour divers noeuds émetteurs du réseau corporel, et différentes stratégies d'acheminement,
- la figure 13 montre un graphique comparatif de la consommation d'énergie pour divers noeuds émetteurs du réseau corporel, et
- la figure 14 montre un graphique comparatif du nombre moyen de sauts pour acheminer un paquet vers le dispositif coordinateur du réseau.

Sur toutes les figures, les mêmes éléments sont référencés par les mêmes numéros.

Dans la suite, on considère la terminologie suivante :

- « BAN » : réseau corporel sans fil (ou « Body Area Networks » en anglais) qui peut être constitué de plusieurs dispositifs, capteurs (ou noeuds) sans fil situés sur un corps mobile, en particulier un corps humain.
- « SNR » : rapport signal à bruit (Signal-to-Noise Ratio en langue anglaise).
- « RSSI » : indicateur de qualité de lien de type matériel, relatif à la puissance du signal reçu (pour « Received Signal Strength Indicator en langue anglaise »).
- « LOI » : indicateur de qualité de lien de type matériel, relatif au niveau des métriques de décision ou d'estimation utilisées pour la démodulation, la synchronisation du signal reçu (pour « Link Quality Indicator » en langue anglaise).
- « NLOS » : situation d'obstruction des liens radios lorsque l'émetteur et le récepteur ne sont pas en visibilité directe (pour « Non-Line-Of-Sight » en langue anglaise).
- « LOS » : propagation d'une onde lorsque l'émetteur et le récepteur sont en visibilité directe (pour « Line-Of-Sight » en langue anglaise).
- « Lien logique » : abstraction d'un lien radio physique qui permet de refléter la connectivité réseau entre une paire de dispositifs sans fil (i.e. lien possible autorisant la transmission et la réception de paquets).
- « Lien fiable par intermittence ou lien fiable sur le court terme » : lien dont la fiabilité/performance varie en raison de la mobilité du corps et du mouvement répétitif des membres, mais présentant de manière intermittente des conditions favorables à la communication (e.g. en termes de réception/perte de paquets).
- « Lien fiable sur le long terme ou lien fiable de manière quasi permanente » : lien dont la fiabilité/performance est quasi permanente au cours du temps, quelles que soient la mobilité ou la posture du corps humain.
- « Durée de contact » : durée pendant laquelle un lien logique est établi, entre une paire de dispositifs sans fil ou noeuds du réseau corporel BAN, et demeure fiable selon un certain critère ou taux de succès applicatif (e.g. en termes de réception/perte de paquets).
- « Durée d'inter-contact » : lorsqu'un lien logique est rompu (suite à une ou plusieurs pertes de paquets), la durée d'inter-contact correspond au temps nécessaire avant que le lien ne se rétablisse à nouveau.
- « Topologie ou architecture réseau » : organisation logique des noeuds construite au niveau de la couche réseau pour router/relayer l'information à partir d'un sous-ensemble de liens radio considérés comme étant les plus fiables.

La figure 2 est un schéma montrant un utilisateur U équipé de six dispositifs sans-fil représentant chacun un noeud du réseau corporel, le noeud N1 situé sur le poignet droit, N2 situé sur la cuisse droite, N3 situé sur la hanche gauche, N4 situé dans le dos à l'arrière, N5 situé à l'épaule gauche et N6 situé au niveau de l'oreille droite.

Chacun de ces dispositifs sans-fils N1 à N6 est par exemple équipé d'un capteur de mesure d'une grandeur physique en relation avec le corps de l'utilisateur U.

La nature du capteur et la grandeur physique mesurée dépendent de l'application envisagée du réseau corporel.

En effet, on envisage par exemple des applications de navigation et / ou de positionnement de groupes de personnes mobiles à l'intérieur des bâtiments, ainsi que des services géo-localisés.

Selon un autre exemple, les capteurs mesurent des grandeurs liées au mouvement (e.g. des accéléromètres), par exemple pour le suivi du geste sportif ou pour des applications de divertissement et de jeux.

Selon encore un autre exemple, l'application peut comprendre la détection de posture ou d'attitude, par exemple, pour la rééducation, le suivi de personnes vulnérables ou âgées, et la surveillance de personnes évoluant dans un environnement sinistré (e.g., pompiers à l'intérieur d'un bâtiment en feu, *etc.).*

Il est également envisagé un réseau corporel pour des applications médicales telles que les implants cardiaques, pompes à insuline, surveillance/monitoring des signes vitaux (température, ECG, *etc.).* Dans ce cas, les dispositifs sans-fil comportent par exemple des capteurs mesurant la température, les pulsations du coeur, la pression sanguine, etc.

Selon encore un autre aspect, on envisage des applications de type « *terminal éclaté* » (écrans, claviers, oreillettes non co-localisées).

La présente invention concerne donc tout type d'application mettant en jeu un réseau corporel sans-fil, où des dispositifs sans-fil communiquent entre eux.

Ainsi, chacun des noeuds N1 à N6 peut communiquer avec un autre noeud du réseau pour autant qu'il puisse établir un lien radio, indiqué par des lignes en traits pointillés sur la figure 2.

En effet, en fonction de divers facteurs comme par exemple une puissance de transmission trop faible, un effet de masquage dû à la mobilité de l'utilisateur U et / ou de la distance entre deux noeuds $N_i$ - $N_j$ ($i \neq j$), il se peut qu'un lien radio ne puisse être établi, ou au moins pas de façon permanente.

Les technologies utilisées sont des technologies radio courte portée, par exemple du type Zigbee™, « Bluetooth™ Low Energy (BLE) » ou « IR-UWB » (pour Impulse Radio Ultra WideBand en langue anglaise).

La puissance de transmission associée à ces dispositifs sans-fils est généralement considérée comme faible pour des raisons de prévention sanitaire (par exemple pour minimiser l'impact des ondes radio émises sur la santé, etc.), pour maximiser les performances d'autres réseaux coexistants par exemple de téléphonie, WIFI, etc (pour minimiser les interférences vis-à-vis des autres réseaux présents à proximité du réseau corporel) et/ou du fait de fortes contraintes applicatives propres aux réseaux corporels (par exemple l'absence de maintenance/remplacement des batteries embarquées, la pérennité énergétique du réseau, le faible encombrement physique des noeuds, etc.).

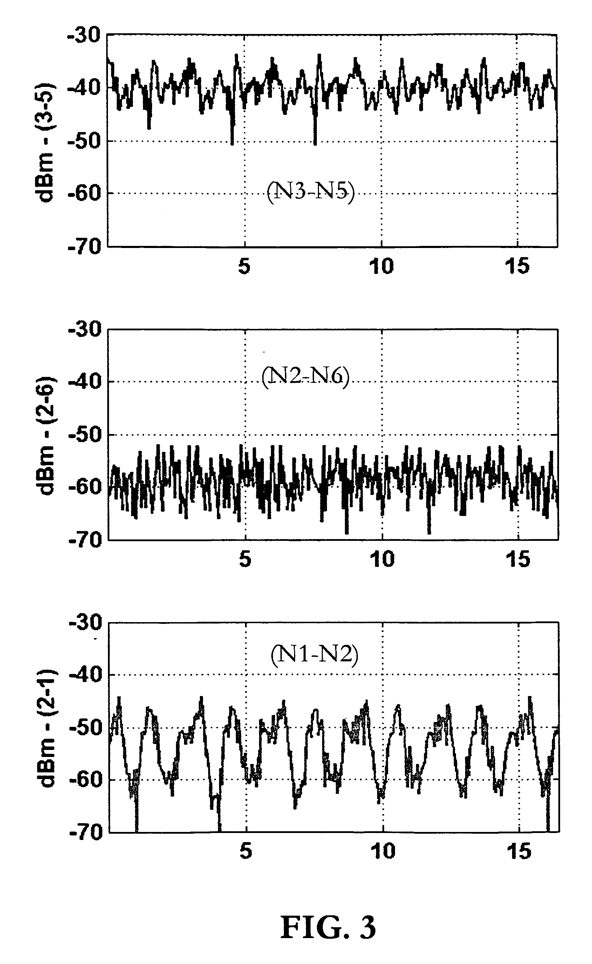

La figure 3 montre sur trois graphiques l'atténuation de la puissance reçue du signal transmis en fonction du temps pour respectivement trois liens radio différents de la figure 2, à savoir N3-N5 pour le graphique supérieur, N2-N6 pour le graphique au milieu et N1-N2 pour le graphique inférieur.

Le graphique supérieur de la figure 3 montre un exemple de l'atténuation de la puissance reçue du signal transmis entre les noeuds N3 et N5, donc la hanche gauche et l'épaule gauche. Ici, les dispositifs associés 3 et 5 sont toujours en visibilité directe (ou « LOS ») avec une faible atténuation de la puissance reçue des ondes radio, ici plus que -40dBm. On considère donc ce lien comme un lien dont les performances (par exemple en termes de taux de livraison des paquets de données, de niveau d'atténuation, de rapport signal à bruit, etc.) sont stables et fiables sur le long terme.

En revanche, sur le graphique du milieu, on voit que le niveau de signal entre les noeuds N2 (situé au niveau de la cuisse droite) et N6 (situé au niveau de l'oreille droite) est fortement dégradé (par exemple le niveau d'atténuation de la puissance reçue (ici inférieur à -60dBm), la dispersion de l'atténuation de la puissance reçue en fonction du temps, etc.), de manière quasi-permanente et/ou instable au cours du temps. Etant donné leur emplacement, cela se comprend, car les dispositifs N2 et N6 sont assez éloignés l'un de l'autre. On obtiendra un comportement similaire par exemple pour le lien entre les noeuds N3 et N4, qui ne sont pas en visibilité directe (ou « NLOS »).

Puis, il y a des liens radio par exemple N1 (situé au niveau du poignet) - N2 (située au niveau de la cuisse droite) dont les performances fluctuent en raison de la mobilité du corps (ou mouvement quasi répétitif ou régulier du corps et/ou des membres, etc.). Le troisième graphique représente par exemple l'effet du balancement de la main le long du corps lors de la marche. Ainsi, les liens radio alternent entre des phases où les performances sont fiables (par exemple dispositifs en visibilité directe ou LOS, avec un faible niveau d'atténuation des ondes radio et donc une forte puissance reçue), et des phases où les performances sont dégradées (par exemple en cas d'obstruction de lien ou NLOS avec une forte atténuation du signal reçu). Il s'agit là de liens radio fiables par intermittence, oscillant entre -45dBm et -70dBm que l'on propose d'évaluer et/ ou de qualifier afin de pouvoir les utiliser pour des transmissions de messages, alors que ces liens-là ne seraient habituellement pas utilisés dans les réseaux corporels de l'état de la technique.

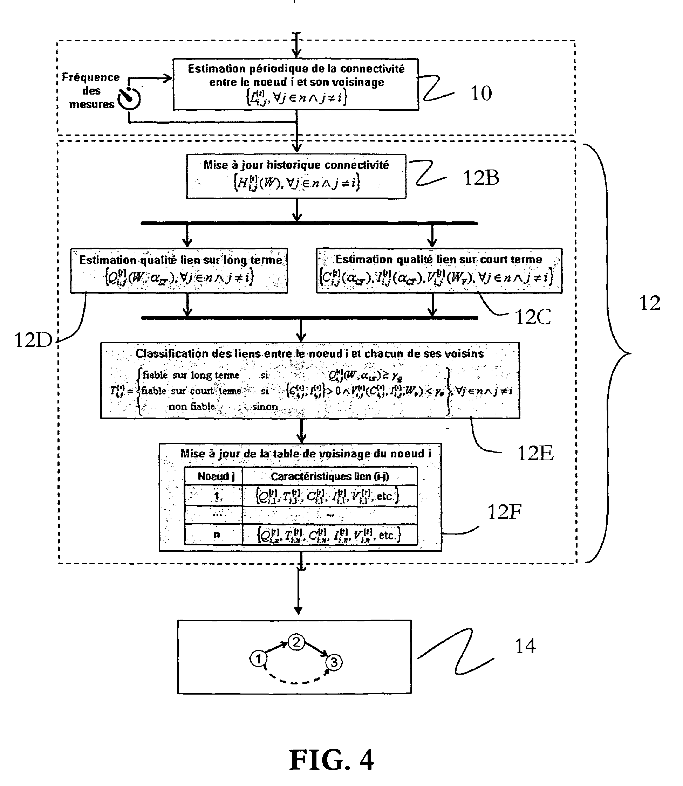

La figure 4 montre un organigramme d'un procédé de transmission de messages au sein d'un réseau corporel sans-fil comprenant plusieurs dispositifs sans-fil susceptibles de communiquer entre eux.

Selon une étape 10, on exploite des messages reçus par au moins un des dispositifs sans-fil $N_i$ - $N_j$ ($i \neq j$, $i,j$ étant des nombres entiers naturels) et on mesure la qualité instantanée des liens radios ($1_{i,j}$) correspondant et on estime des durées pendant et entre lesquelles un lien radio est fiable. On calcule un indicateur de fiabilité des durées estimées et on classifie les liens radio en fonction de l'indicateur de fiabilité en au moins deux catégories ($T_{i,j}$) dont une catégorie relative aux liens radio fiables par intermittence tenant compte de la mobilité des dispositifs sans fil du réseau corporel. Cette étape 12 est détaillée sur la figure avec des sous-étapes qui seront précisées plus loin.

Enfin, lors d'une étape 14, pour la transmission des messages à travers le réseau corporel, on achemine les messages via un lien radio « fiable par intermittence » pendant une période de temps pour laquelle ce même lien radio est considéré comme fiable, c'est-à-dire que l'on tient compte des résultats de classification pour le routage dans le réseau corporel. Cela permet une optimisation du trafic du réseau corporel, par exemple en termes de consommation d'énergie, de rapidité d'acheminement des messages, de latence etc.

Concernant l'étape 10, les messages échangés entre les dispositifs sans-fils N1 à N6 par exemple sont des messages de service (i.e. utiles à l'organisation et à la bonne gestion du réseau), des messages de données ou une combinaison de ces messages.

Par ailleurs, on envisage l'échange de ces messages de façon périodique, pseudopériodoque ou selon une fréquence variable.

On réalise par exemple des échanges par envoi de messages spécifiques du type « hello » à des instants prédéfinis pour chacun des dispositifs sans-fil N1 à N6. Ainsi, le dispositif sans-fil récepteur connaît le moment où il doit recevoir un message et la provenance de ce message (i.e. quel est le dispositif sans-fil émetteur). L'absence de réception peut être qualifiée comme un « lien radio rompu ou inexistant ».

Pour exploiter les échanges on envisage divers scénarios. Selon un premier exemple, on exploite une communication directe entre deux dispositifs sans fil afin d'estimer la qualité du lien radio. Dans ce cas, il s'agit d'une communication point-à-point de type « *Unicast* » et dont l'un des dispositif est le destinataire.

Selon un autre exemple, on exploite une communication directe de type « Broadcast » afin d'estimer la qualité du lien radio vis-à-vis de l'émetteur. Dans ce cas, tous les dispositifs sans fil récepteurs sont les destinataires de cette communication.

Selon un troisième exemple, un dispositif sans fil mettant en oeuvre la présente invention écoute le trafic ou les échanges de données qui transitent en clair sur le canal de communication afin d'estimer la qualité des liens radio entre deux autres dispositifs sans fil. Un accusé de réception échangé permet de signaler la bonne réception du message.

Selon un quatrième exemple, on écoute le trafic ou les échanges de données qui transitent en clair sur le réseau corporel afin d'estimer la qualité des liens radio entre des paires de dispositifs sans fil.

Selon un premier mode de réalisation, on mesure la qualité instantanée des liens radio en mesurant au moins une métrique radio telle que RSSI, LQI, ou SNR.

Selon un mode alternatif, on mesure la qualité instantanée des liens radio en mesurant un paramètre de connectivité, c'est-à-dire, en déterminant seulement si le paquet transmis a été reçu puis démodulé (après détection d'une synchronisation correctement réalisée), et ce, indépendamment de la qualité de cette démodulation. Pour ce faire, on peut déterminer le paramètre de connectivité et / ou l'indicateur radio de façon binaire ayant une valeur « 1 » ou une valeur « 0 » $L_{i,j}^{[t]} = \{0,1\}$  pour tout instant *t*, entre un noeud *i* et *j*, $i \neq j$.

L'indicateur radio peut être défini comme suit :

$$L_{i,j}^{[t]} = \begin{cases} 1 & \text{si l'indicateur radio est supérieur ou égal à un seuil} \\ 0 & \text{si l'indicateur radio est inférieur à un seuil} \end{cases}$$

Le paramètre de connectivité est par exemple un taux de réception de paquets, c'est-à-dire le nombre de paquets reçus par unité de temps.

Ce paramètre de connectivité peut être défini comme suit :
A tout instant *t*, l'état de la connectivité entre un noeud N *i* et N *j*, $i \neq j$,

est  $L_{i,j}^{[t]} = \{0,1\}$ , où

$$L_{i,j}^{[t]} = \begin{cases} 1 & \text{si le lien est actif par exemple si un échange de message a abouti} \\ 0 & \text{si le lien est inactif par exemple si un échange de message a échoué.} \end{cases}$$

Etant donné la détermination binaire du paramètre de connectivité et de l'indicateur radio, on peut dans un même réseau corporel utiliser les deux paramètres à la fois.

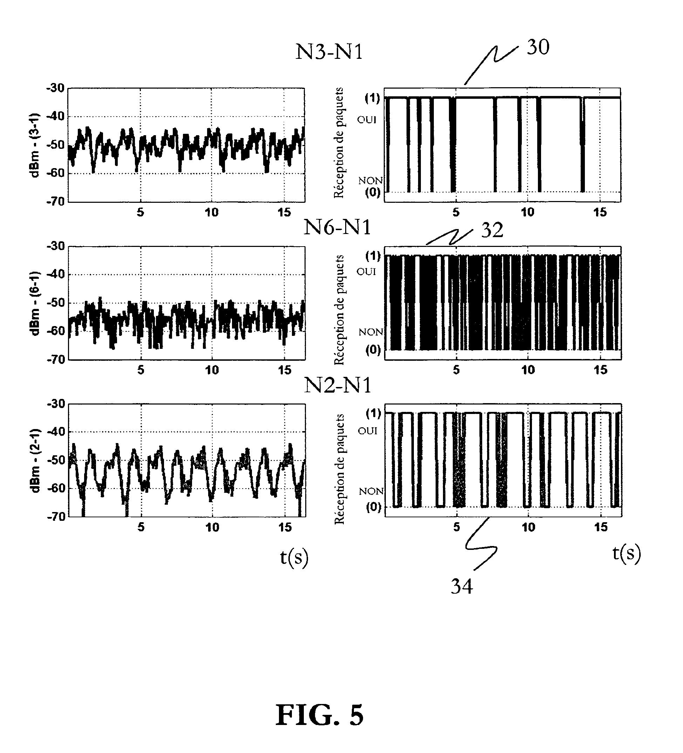

La figure 5 montre pour les trois liens N3-N1, N6-N1 et N2-N1 de la figure 2 respectivement un graphique de niveau de signal / niveau d'atténuation de la puissance reçue en fonction du temps (graphique de gauche) et un graphique du paramètre de connectivité (graphique de droite), défini à partir de la bonne réception (ou non) des paquets.

Il s'agit donc d'un exemple de mesures de qualité instantanée de liens d'un dispositif sans-fil N1 vis-à-vis de ses trois noeuds voisins N3, N6 et N 2 (voir Figure 2).

Ce dispositif sans-fil N1 exploite par exemple un échange périodique de trafic de contrôle de type « hello » afin de déterminer, à chaque instant et de manière périodique, l'état de la connectivité vis-à-vis des noeuds présents dans son voisinage (i.e. les dispositifs 3, 6 et 2 qui sont présents à portée de communication).

Les graphiques de la figure 5 sont issus d'une campagne expérimentale à 2.45GHz de dispositifs sans-fils attachés à un corps mobile (selon le schéma de la Figure 2) avec un échange périodique d'un trafic de contrôle entre les différents noeuds (un paquet hello envoyé toutes les 60ms par chaque noeud). Il s'agit d'une campagne de sondage du canal avec abstraction d'une couche physique de type Bluetooth Low Energy à partir des valeurs expérimentales du SNR.

Comme on peut le constater sur la figure 5, grâce à la connaissance de la fréquence d'envoi des différents paquets « hello », le dispositif N1 est capable de mesurer périodiquement l'état de sa connectivité vis-à-vis de son voisinage.

Le graphique de connectivité 30 entre les dispositifs N3 et N1 montre une connectivité quasi permanente et fiable, interrompue seulement par quelques pertes de paquets.

Le graphique de connectivité 32 entre les dispositifs N6 et N1 montre une connectivité quasi aléatoire, avec des pertes importantes de paquets.

Le graphique de connectivité 34 entre les dispositifs N2 et N1 montre une bonne connectivité sur certaines périodes interrompues par des périodes de pertes importantes de paquets.

Ainsi, la connectivité associée aux différents liens radio varie au cours du temps, et dépend principalement de l'état du canal (graphiques de gauche), où les phénomènes de propagation (par exemple l'atténuation du signal, les effets de masquage des ondes radio, etc.) et de mobilité du corps humain impactent directement les performances des échanges de données entre les noeuds, en termes de bonne réception ou de perte des paquets « hello ».

Ces mesures sont ensuite exploitées dans l'étape 12 (voir figure 4) pour classifier les différents liens radio à l'issue de cette première étape 10 afin de caractériser les performances des différents liens radio à partir des informations instantanées et passées de connectivité.

Pour pouvoir exploiter les liens radio fiables par intermittence, il est nécessaire de les classifier selon au moins les deux catégories évoquées ci-dessus. Bien entendu, une classification plus fine avec plusieurs niveaux de liens radio fiables par intermittence pourrait être adoptée.

Pour cela, on détermine lors de l'étape 12 à partir du paramètre de connectivité pour respectivement deux dispositifs de lien radio sans-fil, des durées de contact et d'inter-contact et on détermine la présence de motifs répétitifs de contact par intermittence lors d'une sous-étape 12A.

La durée de contact, $\hat{C}_{i,j}^{[t]}$, peut par exemple être calculée comme la durée d'une séquence binaire

$$L_{N+M} = \left\{ L_{i,j}^{[1]}, L_{i,j}^{[2]}, ..., L_{i,j}^{[N+M]} \right\} \in \{1\}^N \ et \ \{0\}^M$$ et telle que $\dfrac{N-M}{N} \geq \gamma_F$, où $\gamma_F$ est un seuil prédéterminé de fiabilité du lien radio. Cela revient à déterminer la durée de contact comme étant la période de temps pendant laquelle le taux de réception des paquets associé à ce lien est supérieur ou égal au seuil $\gamma_F$, i.e. $PRR_{i,j}^{[t]}(\hat{C}_{i,j}^{[t]}) \geq \gamma_F$.

$\gamma_F$ est choisi en fonction des exigences de transmission des messages selon les applications.

En effet, ce seuil dépend principalement des contraintes et/ou des besoins des applications. Ainsi ce seuil peut être très élevé pour des applications critiques (e.g. applications médicales, etc.) ou plus relâché pour des applications de types jeux, navigation, etc.

Par exemple, pour une application médicale ou de surveillance, le seuil $\gamma_F$ peut être choisi supérieur ou égal à 95%. On donne donc priorité à la réussite de transmission des messages.

En revanche, pour un réseau corporel sans-fil destiné au jeu ou à la navigation, ce seuil $\gamma_F$ peut être abaissé à 80%. En effet, dans ce type d'application, la perte d'un paquet est moins critique.

La durée d'inter-contact est définie d'une manière similaire à la durée de contact. Cette durée d'inter-contact

$\hat{I}_{i,j}^{[t]}$, peut être calculée comme la durée d'une séquence binaire $L_{N+M} = \left\{ L_{i,j}^{[1]}, L_{i,j}^{[2]}, ..., L_{i,j}^{[N+M]} \right\} \in \{1\}^N$ et $\{0\}^M$ et

telle que $N \leq \gamma_{UP}$, où $\gamma_{UP}$ est le nombre maximal de « 1 » toléré durant la période d'inter-contact.

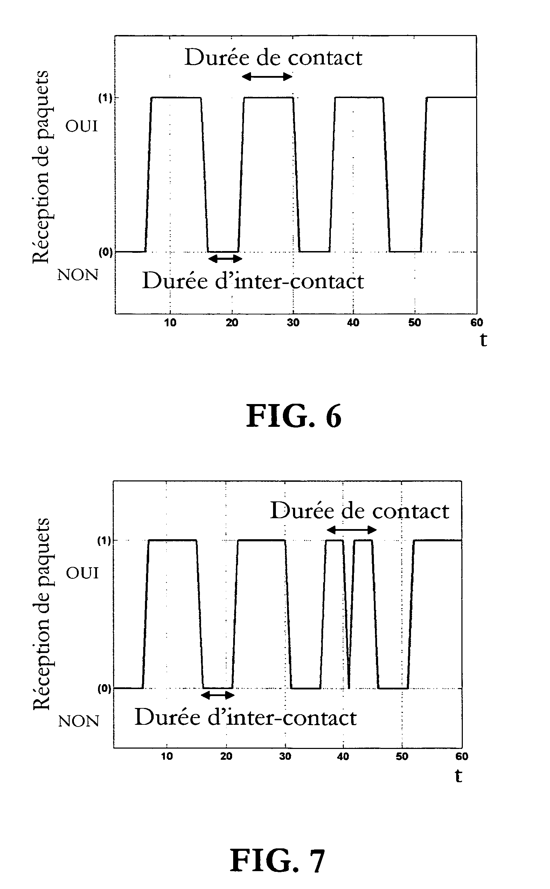

Les figures 6 et 7 montrent des exemples d'évolution de la connectivité $L_{i,j}^{[t]} = \{0,1\}$ en fonction du temps, où les durées de contact et d'inter-contact sont interprétées à partir de l'information de connectivité, respectivement selon une approche déterministe et une approche probabiliste.

Comme on le voit sur les figures 6 et 7, la durée de contact d'un lien radio correspond au temps pendant lequel un lien est établi et demeure fiable (ou connecté). Dès qu'un lien est rompu (suite à une ou plusieurs pertes de paquets), la durée d'inter-contact correspond au temps nécessaire avant que le lien ne se rétablisse de nouveau.

Vu les spécificités inhérentes aux réseaux corporels sans-fil, notamment liées à la mobilité quasi périodique et répétitive du corps ou des membres, ces durées de contact et d'inter-contact sont censées varier de manière plus ou moins « régulière » ou périodique au cours du temps en fonction, notamment, de la vitesse de mobilité ou de la posture du corps porteur.

Ainsi, pour un lien radio donné, si les durées évaluées de contact et d'inter-contact évoluent de manière stable au cours du temps, il est fort probable que ce lien soit de type fiable sur le court terme (et pour peu que l'instant soit judicieusement choisi), c'est-à-dire fiable par intermittence.

Dans le cas contraire, si les durées évaluées de contact et d'inter-contact sont très variables, d'une évaluation à une autre, alors il est fort probable que ce lien ne soit pas fiable.

Plusieurs autres algorithmes peuvent être envisagés pour l'estimation de ces durées de contact et d'inter-contact à partir d'un historique de mesures de connectivité entre une paire de noeuds.

Par exemple, les techniques de reconnaissance de formes (ou motifs) et/ou d'apprentissage automatique (e.g. modèles de Markov cachés, réseau de neurones, etc.) peuvent être mise en oeuvre afin de déterminer, à partir des informations de connectivité brutes (l'historique H) la présence d'un motif répétitif de connexion/déconnexion du lien.

Pour procéder à la classification, il faut donc évaluer d'une part ces durées de contact et d'inter-contact, et d'autre part la stabilité des évaluations au cours du temps afin de détecter, parmi l'ensemble des liens radio un sous-ensemble de liens fiables sur le court terme ou par intermittence (connectivité qui alterne de manière périodique et répétée entre des états où le lien est fiable et d'autres états où le lien n'est pas fiable).

Etant donné une fenêtre d'observation temporelle de taille $W$, à chaque instant $t$, chaque dispositif sans fil N$i$ dispose, à l'issue de la sous-étape 12A, d'un historique $H_{i,j}^{[t]}(W) = \left\{ L_{i,j}^{[t-W+1]} \ldots L_{i,j}^{[t-1]} L_{i,j}^{[t]} \right\}$ représentant l'état de la connectivité (mesurée durant la période de temps passée $W$) vis-à-vis de chaque dispositif N $j$, $\forall\, j \in n \wedge j \neq i$, présent à portée de communication (voir sous-étape 12B).

Idéalement, puisque les performances d'un lien radio sont fortement corrélées aux conditions d'obstruction, la durée de contact $\hat{C}_{i,j}^{[t]}$ (respectivement, durée d'inter-contact $\hat{I}_{i,j}^{[t]}$ ) peut être calculée en comptant le nombre successif de « 1 » (respectivement « 0 ») dans la séquence binaire $H_{i,j}^{[t]}(W)$ (approche déterministe de la figure 6).

Cependant, en présence d'un évanouissement local du signal, des pertes de paquets sont également possibles même si le lien est non-obstrué (condition LOS) et que la puissance de transmission est suffisante.

Afin de mieux prendre en compte l'aspect aléatoire du canal radio, nous adoptons une approche probabiliste, comme illustré sur la Figure 7, permettant de tolérer la perte d'un nombre prédéfini de paquets lors d'une période de contact.

Pour estimer les durées finales de contact et d'inter-contact (à partir des durées estimées courantes et passées), on applique lors d'une sous-étape 12C une technique de lissage exponentiel :

$$C_{i,j}^{[t]}(\alpha_{CT}) = \alpha_{CT} \times C_{i,j}^{[t-1]} + (1 - \alpha_{CT}) \times \hat{C}_{i,j}^{[t]}$$

$$I_{i,j}^{[t]}(\alpha_{CT}) = \alpha_{CT} \times I_{i,j}^{[t-1]} + (1 - \alpha_{CT}) \times \hat{I}_{i,j}^{[t]}$$

Où $\hat{C}_{i,j}^{[t]}$ (respectivement $\hat{I}_{i,j}^{[t]}$ ) est l'estimation instantanée (ou courante) de la durée de contact (respectivement inter-contact) ; $C_{i,j}^{[t]}$ (respectivement $I_{i,j}^{[t]}$ ) est l'estimation finale à l'instant $t$ de la durée de contact (respectivement inter-contact) ; et enfin $\alpha_{CT}$ est un facteur d'oubli qui permet de donner plus ou moins d'importance aux estimations passées.

Enfin, afin d'apprécier la stabilité des différentes estimations, on évalue le coefficient de variation glissant, $V_{i,j}^{[t]}$, ou MCV (pour « *Moving Coefficient of Variation* » en langue anglaise) sur une fenêtre de taille $W_V$. L'objectif de ce coefficient est d'apprécier la dispersion (ou inversement, stabilité) des $W_V$ évaluations passées des durées de contact et d'inter-contact. Il est défini comme étant le rapport entre l'écart type et la moyenne des estimations sur une fenêtre glissante de taille $W_V$.

Ainsi, si ce coefficient de variation glissant $V_{i,j}^{[t]}$ est inférieur à un certain seuil, $\gamma_V$, alors les estimations sont considérées comme stables au cours du temps, indiquant ainsi la présence d'un lien fiable par intermittence, avec des périodes régulières et périodiques de contact et d'inter-contact. Sinon, si le coefficient de variation glissant est supérieur à $\gamma_V$, alors les estimations sont considérées comme trop variables, et le lien correspondant est classifié comme étant non fiable.

Selon des variantes envisagées, on peut aussi évaluer la stabilité d'une série d'estimations (écart type, écart moyen, variance, moyenne glissante, coefficient de variation, etc).

Pour l'évaluation de qualité de liens sur le long terme ou fiable de façon quasi permanente, on utilise par exemple lors de la sous étape 12D un estimateur WMEWMA (pour « *Window Mean with Exponentially Weighted Moving Average* » en langue anglaise, c'est-à-dire un estimateur de fenêtre moyen avec une moyenne glissante pondérée de façon exponentielle).

A chaque instant $t$, et à partir de l'historique $H_{i,j}^{[t]}(W)$ mesuré sur une période de temps $W$ et vis-à-vis d'un noeud voisin N $j$, l'estimateur WMEWMA commence par évaluer le taux de réception des paquets ou PRR (pour « *Packet Reception Ratio* » en langue anglaise) :

$$PRR_{i,j}^{[t]}(W) = \frac{1}{W} \sum_{k=t-W+1}^{t} L_{i,j}^{[k]}$$

Enfin, l'estimateur WMEWMA peut être calculé selon une moyenne glissante exponentielle pour l'estimation de la qualité du lien radio sur le long terme :

$$Q_{i,j}^{[t]}(W, \alpha_{LT}) = (1 - \alpha_{LT}) \times Q_{i,j}^{[t-1]} + \alpha_{LT} \times PRR_{i,j}^{[t]}(W)$$

Où $Q_{i,j}^{[t]}$ est l'estimation ou l'évaluation, à l'instant $t$, de la qualité du lien (i-j) entre les noeuds ou dispositifs sans-fil (Ni-Nj) sur le long terme ; $\alpha_{LT}$ est un facteur d'oubli permettant de donner plus ou moins d'importance aux estimations passées ; et enfin $W$ est la taille de la fenêtre d'observation.

Ainsi, on peut déterminer lors de la sous-étape 12E avec précision le type de chaque lien radio $(T_{i,j}^{[t]})$ selon trois classes principales : les liens fiables sur le long terme ou fiables de façon quasi permanente grâce à l'estimateur WMEWMA, les liens fiables par intermittence ou fiables sur le court terme grâce au coefficient de variation glissant, $V_{i,j}^{[t]}$ et les liens non fiables.

A partir de ces différentes informations, , une table de voisinage du dispositif sans-fil est mise à jour lors d'une sous-étape 12F. Cette table est par exemple enregistrée dans une mémoire contenant la liste des différents noeuds / dispositifs sans-fil voisins présents à portée de communication avec une évaluation du type (fiable, fiable par intermittence ou non fiable) ainsi que des propriétés de chaque lien radio.

Ces tables de voisinages mémorisées dans chaque dispositif sans-fil peuvent être par la suite exploitées par :

1) un algorithme de routage opportuniste pour le calcul d'un chemin optimal vers une destination en fonction de certaines contraintes applicatives (par exemple énergie, délai) ;

2) un algorithme de relais opportuniste et adaptatif pour prédire l'apparition de certains liens radio vers les noeuds de destination ; ou par

3) un algorithme de radiolocalisation pour un ordonnancement optimal des mesures de distance relatives à réaliser entre les différents noeuds du réseau, en fonction de la disponibilité ou non des liens radio.

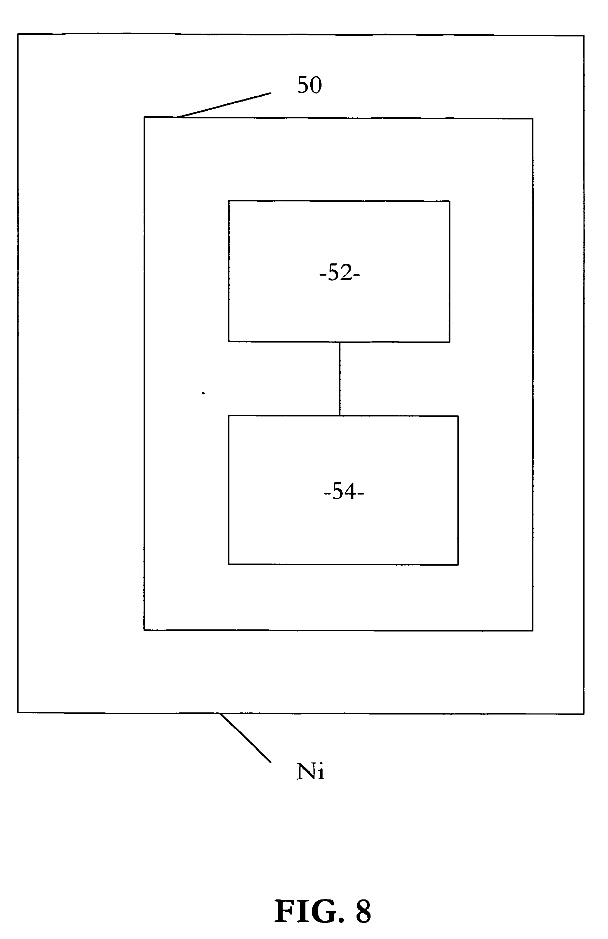

L'invention concerne également un dispositif 50 de transmission de messages à travers un réseau corporel sans-fil comprenant plusieurs dispositifs sans-fil formant un réseau corporel et susceptibles de communiquer entre eux. La figure 8 montre un schéma synoptique d'un tel dispositif 50 selon l'invention pour la mise en oeuvre du procédé de la figure 4.

Ce dispositif de transmission 50 comprend d'une part un dispositif 52 configuré pour évaluer la qualité des liens radio pour un réseau corporel sans-fils, c'est à dire pour réaliser les étapes 10 et 12, et d'autre part des moyens 54 configurés pour acheminer ou relayer les messages via un lien radio fiable par intermittence pendant une période pendant laquelle le lien radio fiable par intermittence est considéré comme fiable. Les moyens 54 sont reliés aux moyens d'évaluation de la qualité des liens radio 52 et exploitent les résultats de ces moyens 52.

Concernant le dispositif 52 d'évaluation de la qualité des liens radio, celui-ci est configuré pour la mise en oeuvre des étapes 10 et 12 du procédé décrit ci-dessus. Il s'agit par exemple d'une unité intégrée dans un ou plusieurs dispositif sans-fil N1 à N6 du réseau corporel BAN avec un processeur et des mémoires configurées pour réaliser les diverses étapes du procédé ci-dessus et pour mémoriser les résultats par exemple dans une table de voisinage. Bien entendu, cette unité peut être spécifique par rapport à une unité de capteur et ou de télécommunication sans fil ou il peut s'agir du processeur et des mémoires du dispositif sans-fil déjà présent.

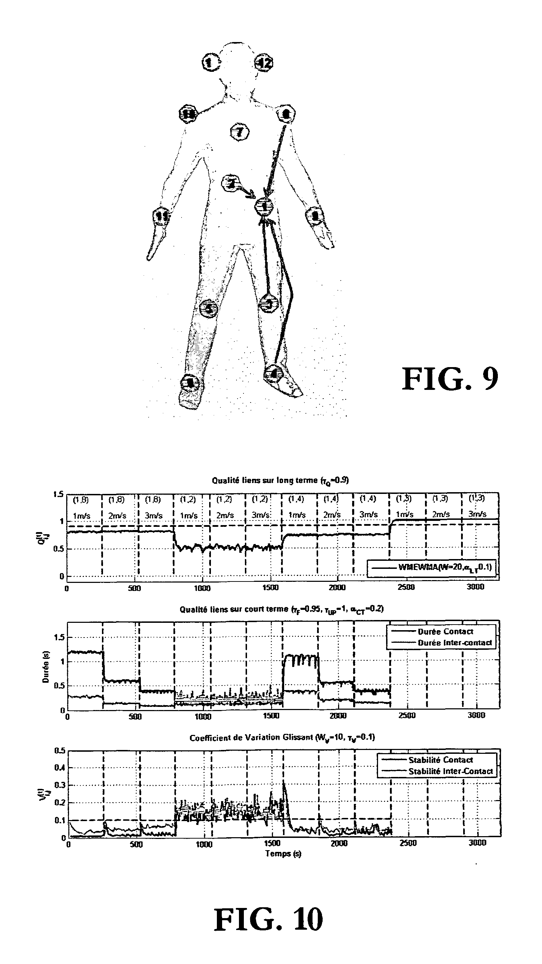

On se réfère maintenant aux figures 9 et 10.

Sur la figure 9 est représenté un utilisateur U avec douze dispositifs sans-fil numérotés N1 à N12 et situés à divers endroits du corps.

On s'intéresse en particulier au dispositif sans-fil ou noeud N1 qui est le noeud concentrateur vers lequel tous les messages doivent être acheminés pour ensuite communiquer par exemple avec un réseau externe 6 (non représenté sur cette figure).

La figure 10 montre trois graphiques dont le graphique supérieur illustre l'estimateur de qualité sur le long terme $Q_{i,j}^{[t]}$, le graphique au milieu les durées de contact et inter-contact définies ci-dessus, et le graphique inférieur montre le coefficient de variation glissant, $V_{i,j}^{[t]}$.

Chaque graphique est sous-divisé pour montrer l'évolution de ces valeurs en fonction du temps, respectivement pour les liens entre les noeuds / dispositifs sans-fil N1-N8, N1-N2, N1-N4 et N1-N3 (voir aussi figure 9, ceci suivant trois différentes vitesses de mobilité (1m/s, 2m/s et 3m/s).

Dans cet exemple, il est à noter que chaque intervalle de temps (12 intervalles au total) correspond à une simulation impliquant un lien radio ainsi qu'une vitesse de mobilité du corps bien déterminée. Chaque intervalle a une durée de 264 secondes, correspondant à un échange de 4400 paquets « hello » entre les dispositifs sans-fil avec une fréquence d'un paquet hello par 60ms.

Durant les trois premiers intervalles de temps, on considère les mesures de qualité correspondant au lien radio (N1-N8) (considéré comme fiable sur le court terme) suivant 3 vitesses de mobilité (de 1m/s à 3m/s).

Sur la figure 10, un seuil de 90% a été défini pour décider si un lien est fiable sur le long terme. On remarque qu'avec l'estimateur *WMEWMA*, $Q_{i,j}^{[t]}$, la qualité du lien est d'environ 80%, et l'évaluation de la qualité des liens sur le court terme s'adapte bien à la mobilité du BAN et montre des durées de contact et d'inter-contact, avec un coefficient de variation très faible (<0.1). Ce lien est dans ce cas classifié comme étant « fiable par intermittence » ou « fiable sur le court terme ».

Durant les trois intervalles suivants, on considère les mesures de qualité correspondant au lien radio (N1-N2) (le noeud N2 étant situé à l'arrière du corps par rapport au noeud 1). On remarque que la qualité du lien sur le long terme est en moyenne égale à 50%, et que les estimations de durées de contact et d'inter-contact ne sont pas stables, avec un coefficient de variation très élevé. Le lien est donc classifié comme « non fiable ».

Considérons ensuite les mesures relatives aux liens radio (N1-N4) (lien fiable par intermittence ou sur le court terme) et (N1-N3) (lien fiable sur le long terme). On constate encore une fois que le procédé selon l'invention s'adapte bien aux différentes vitesses ainsi qu'aux différents types des liens, et permet de déterminer, à la fois sur le long et le court terme, les performances des différents liens radio, et de les classifier en fonction de leurs caractéristiques en vue de les exploiter ultérieurement par des protocoles de plus haut niveau (e.g. routage, relayage, prédiction, etc.).

L'étapes 14 du procédé de la figure 4 consistent à exploiter les différentes tables de voisinage, déterminées précédemment, en vue d'une part de prédire sur la base des tables de voisinage de façon probabiliste l'apparition des liens radio fiables par intermittence et d'utiliser ces liens pour la transmission des messages lorsqu'ils existent en vue d'améliorer les performances ainsi que la robustesse des protocoles de plus haut niveau, notamment face à la mobilité du corps et aux phénomènes de propagation autour ou à forte proximité du corps humain (e.g. masquage et obstruction des liens radio, propagation multi-trajets, absorption des ondes radio par le corps humain, etc.).

Ainsi, chaque dispositif sans-fil N $i$, $\forall\ i \in n$, du réseau corporel, dispose localement d'une table de voisinage contenant, pour chacun de ses noeuds voisins N $j$ (ou noeuds présents à portée de communication), $\forall\ j \in n \wedge j \neq i$, d'un n-tuplet $\left\{Q_{i,j}^{[t]}, T_{i,j}^{[t]}, C_{i,j}^{[t]}, I_{i,j}^{[t]}, V_{i,j}^{[t]}, \right\}$, où : $Q_{i,j}^{[t]}$ est la qualité du lien (Ni-Nj) sur le long terme, $T_{i,j}^{[t]}$ est le type du lien (Ni-Nj) : fiable sur le long terme, fiable sur le court terme, ou non fiable, $C_{i,j}^{[t]}$ est la durée de contact estimée pour le lien (Ni-Nj), $I_{i,j}^{[t]}$ est la durée d'inter-contact estimée pour le lien (Ni-Nj), $V_{i,j}^{[t]}$ est le coefficient de variation glissant représentant la dispersion (ou la stabilité) associée aux estimations $C_{i,j}^{[t]}$ et $I_{i,j}^{[t]}$.

Ces différentes informations sont ainsi principalement exploitées par les protocoles de plus haut niveau pour :

- identifier la présence de liens fiables sur le court terme en vue de maximiser les performances des protocoles de routage et de relayage en termes de consommation d'énergie, de latence, de taux de livraison des données, etc, et
- maximiser les performances des algorithmes de localisation coopérative en permettant un ordonnancement optimal des mesures de distance entre les noeuds, grâce à la prédiction d'apparition et/ou de disparition des liens fiables sur le court terme (à partir des informations $T_{i,j}^{[t]}, C_{i,j}^{[t]}, I_{i,j}^{[t]}$ et $V_{i,j}^{[t]}$).

Pour la transmission des messages, ayant la connaissance de fiabilité des liens radio du réseau corporel, on peut maintenant acheminer les messages via un lien radio fiable par intermittence pendant une période de temps pour laquelle le lien radio fiable par intermittence est considéré comme fiable.

En fonction de l'application envisagée, on favorise la transmission d'un message via un lien radio fiable par intermittence pour optimiser au moins un des paramètres du groupe suivant : consommation d'énergie, la latence, le taux de livraison des données.

De plus, selon certaines applications, on peut ordonnancer des mesures de distance entre les dispositifs sans-fil en tenant compte de la prédiction d'apparition et/ou de disparition des liens fiables par intermittence.

Par la suite, on donne un exemple illustratif.

Les divers tables de voisinage au niveau de noeuds / dispositifs sans-fil peuvent être exploités de différentes manière en fonction de l'application et d'un paramètre / critère de priorité que l'on veut donner pour la transmission des messages.

En effet, à partir des différentes informations liées à la réception ou non de paquets de données, chaque noeud maintient dans sa table de voisinage la liste des noeuds sans-fil à portée de communication et calcule une métrique de coût pour chaque lien radio correspondant. Cette métrique de coût dépend principalement des contraintes applicatives les plus importantes, comme par exemple, le taux de livraison des données, le délai d'acheminement des données ou la consommation d'énergie, et reflète le degré de fiabilité de chaque lien radio ou séquence de liens (ou chemin).

Étant donné un réseau corporel constitué de $n$ dispositifs sans-fil, le nombre maximal de liens (orientés) est n(n - 1), et chaque lien logique $l_{i,j}$, existant entre une paire de noeuds N $i$ et N $j$, $i \neq j$, est doté d'une métrique de coût que l'on note $e_{i,j}^{[t]}$.

Deux principales formulations de cette métrique de coût peuvent être envisagées selon les contraintes applicatives :

1) la métrique du PDR (pour « Probability Delivery Ratio » en anglais, c'est à dire la ratio de délivrance probable) pour les applications critiques non tolérantes aux pertes de paquets ; ou
2) la métrique du ANR (pour « Average Number of Retransmissions » en anglais pour nombre moyen de retransmissions) pour les applications fortement contraintes en termes d'énergie et de délai.

Pour l'exemple non limitatif qui sera donné par la suite, on considéra la métrique du PDR. La métrique ANR peut s'appliquer de manière similaire.

La métrique du PDR (« Probability Delivery Ratio ») est définie comme le taux de livraison des données (ou la qualité du lien sur le long terme), et peut être déterminée grâce à un estimateur de qualité de liens radio. En considérant l'estimateur WMEWMA, la métrique de coût associée à chaque lien $l_{i,j}$, $i \neq j$, est définie comme suit : $e_{i,j}^{[t]} = Q_{i,j}^{[t]}$, où $Q_{i,j}^{[t]}$ est la qualité estimée du lien $l_{i,j}$ sur le long terme (grâce à WMEWMA).

Dans ce cas, à chaque instant $t$ et étant donné un noeud source $i$ et un noeud destination $j$, le chemin optimal, $R_{i,j}$, reliant ces deux noeuds est déterminé comme étant le chemin qui maximise la fiabilité de bout-en-bout des communications en termes de livraison des paquets de données, i.e. $R_{i,j} = \max_R \prod_{l \in R} e_l^{[t]}$. Le chemin $R_{i,j}$ ainsi calculé, et se composant éventuellement par un ensemble de lien du réseau, est le chemin qui maximise le coût global (ou de bout-en-bout) en termes de fiabilité des communications.

Le calcul de ce chemin peut être réalisé au moyen d'un algorithme modifié de calcul du plus court chemin de type Dijkstra, bien connu de l'homme de l'art.

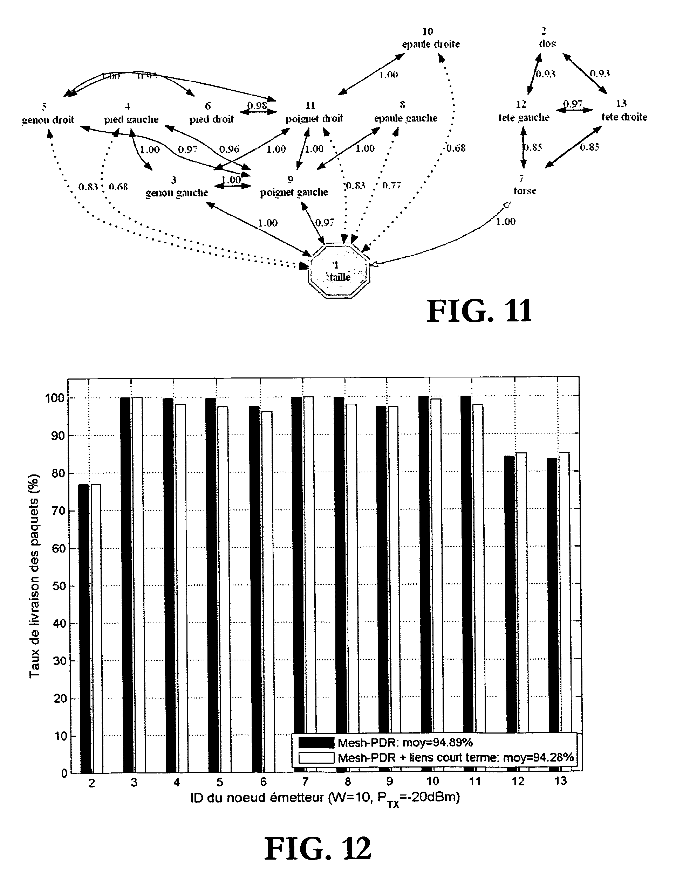

Afin d'illustrer l'impact de cette métrique de routage sur les performances obtenues avec un routage opportuniste utilisant les liens fiables par intermittence, on présente sur la Figure 11 un exemple d'application.

La Figure 11 représente le graphique de connectivité associé au BAN de la Figure 9, où le noeud N1 est considéré comme le coordinateur / concentrateur du réseau, et où chaque lien est doté d'une métrique de coût relative au taux de livraison des données (ou $Q_{i,j}^{[t]}$ qualité du lien sur le long terme et reflétant la fiabilité des communications).

On suppose que le noeud N5 souhaite envoyer un paquet de données vers le coordinateur N1.

Sur ce graphique, les liens $l_{i,j}$ en trait plein représentent des liens radio fiables à long terme et en trait pointillés les liens fiables par intermittence. Les chiffres à côtés des traits expriment le coût du lien radio, à savoir $e_{i,j}^{[t]}$.

Si on considère la métrique de routage du PDR classique n'utilisant que les liens fiables à long terme, le chemin optimal est celui qui maximise la probabilité de livraison des données.

Par conséquent, le chemin optimal sélectionné est de longueur 3, $R_{5,1} = \{l_{5,11}, l_{11,3}, l_{3,1}\}$ avec un coût total (ou fiabilité de bout-en-bout associée au chemin global) égal à : $\prod_{l \in R_{5,1}} e_l^{[t]} = e_{5,11}^{[t]} . e_{11,3}^{[t]} . e_{3,1}^{[t]} = 1$.

Ainsi, comme on peut le constater, cette métrique tend à maximiser la probabilité de livraison des données, mais au détriment d'une augmentation significative de la longueur du chemin de routage et, in fine, du délai d'acheminement et de la consommation d'énergie.

Si on considère à la fois les liens radio fiables sur le long terme et ceux fiables par intermittence (c'est-à-dire sur le court terme), on peut encore optimiser le processus de routage.

En effet, grâce à la mise en oeuvre du procédé selon l'invention, le noeud 5 est capable de déterminer la présence d'un chemin fiable et temporaire en direction du coordinateur 1 (par exemple lien en pointillés entre les noeuds N5 et N1 sur la figure 11).

L'algorithme de routage opportuniste ou adaptatif qui en résulte fonctionne comme suit :

Si à un instant donné, ce lien temporaire existe et est détecté par le noeud N5, les données sont directement envoyées au coordinateur N1.

Dans le cas contraire, un chemin est calculé (selon la métrique du PDR), et les données sont relayées de noeud en noeud en direction du coordinateur. Si possible, on favorise donc la transmission des messages via des liens fiables par intermittence quand ils existent et si on peut prédire leur apparition.

On tire donc partie de manière opportuniste de la présence de certains liens radio fiables et stables sur le court terme afin de minimiser la longueur des chemins de routage, et in fine minimiser la consommation d'énergie ainsi que le délai d'acheminement des données.

En variante, si un lien direct de type fiable par intermittence est détecté entre un noeud source et un noeud destination, on peut faire le choix de n'émettre les données que lorsque ce lien apparait ou est présent.

Lorsque ce lien disparait temporairement, le noeud source peut décider de temporiser ses transmissions (même si un chemin alternatif et fiable existe) jusqu'au moment où le lien fiable par intermittence réapparait.

Afin d'évaluer l'apport du procédé selon l'invention par rapport aux architectures de routage conventionnelles, des simulations ont été réalisées.

On considère dans ce cas un trafic de type multipoints-to-point (MP2P) où chaque noeud / dispositif sans-fil du BAN (cf. scénario de la figure 9) envoie périodiquement un paquet de données en direction du coordinateur (noeud N1). Ces paquets de données peuvent par exemple contenir des mesures de distance (e.g. application de type de radiolocalisation) ou des informations relatives au corps humain (e.g. application médicale de monitoring).

On évalue et on compare, dans ce qui suit, deux architectures de communication :

- Une architecture multi-sauts avec le PDR comme métrique de routage (appelée ci-après « *Mesh-PDR* »), où chaque noeud détermine le chemin qui maximise la probabilité de livraison des données de bout-en-bout vers le coordinateur. C'est le schéma classique qui n'utilise que des liens radio fiables sur le long terme (liens en trait plein de la figure 11).
- Une architecture multi-sauts opportuniste ou adaptative avec le PDR comme métrique de routage (appelée ci-après « *Mesh-PDR + liens* « court *ferme* ») où chaque noeud détermine :

    1) un chemin temporaire, fiable et direct vis-à-vis du coordinateur N1 tenant compte des liens radio fiables par intermittence ;
    ou le cas échéant
    2) le chemin qui maximise la probabilité de livraison des données vers le coordinateur N1.

Par ailleurs, on suppose que toutes ces architectures reposent sur un mode de communication sans utilisation de mécanismes de retransmission et un calcul des chemins à partir d'un algorithme de Dijkstra.

On s'intéresse en particulier à trois métriques de performance :

    1) la consommation globale d'énergie ;
    2) le taux de livraison des données calculé comme le rapport entre le nombre de paquets reçus par le coordinateur sur le nombre total de paquets transmis ; et enfin
    3) le délai d'acheminement des données.

La consommation globale d'énergie est calculée proportionnellement au nombre de paquets reçus et transmis par les dispositifs sans fil ainsi qu'aux durées de temps pendant lesquelles ces noeuds sont actifs.

Les résultats de simulation obtenus sont présentés respectivement sur les figures 12, 13 et 14 (la vitesse de mobilité du BAN est de 1m/s et une fréquence d'émission des paquets « *hello* » de 60ms, la puissance de transmission est de -20dBm).

Le taux de livraison des données par noeud est illustré sur la Figure 12. On constate tout d'abord que pour l'architecture « *Mesh-PDR* » (architecture de communication multi-sauts conventionnelle), le taux de livraison des données (des noeuds N2-N13 au coordinateur N1) est en moyenne égal à 94.89%.

L'utilisation du procédé selon l'invention, qui prend en compte les liens radio fiables par intermittence et favorise la transmission des messages via ces liens radio lorsqu'ils apparaissent (« *Mesh-PDR + liens court terme* »), n'engendre pas de baisse de performances significative par rapport à l'architecture de routage conventionnelle, avec un taux moyen de livraison des données de 94.28%.

En revanche, on constate des différences lorsque l'on analyse ces architectures sous l'angle de la consommation d'énergie.

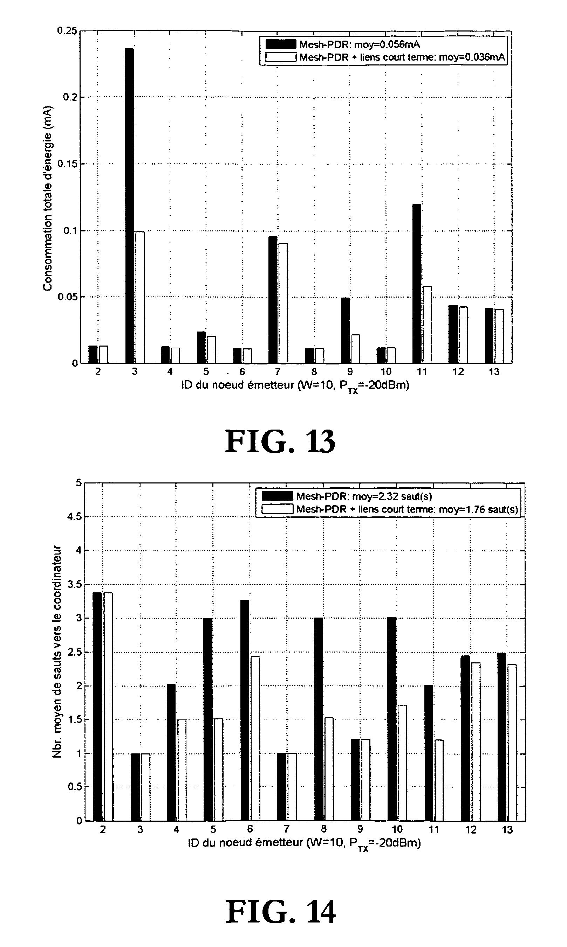

Cette consommation d'énergie par noeud est illustrée sur la Figure 13. Nous remarquons tout d'abord que cette dernière est non uniforme. En effet, dans les architectures multi-sauts, certains noeuds, en plus de transmettre leurs propres paquets, sont susceptibles de relayer les paquets de ses noeuds voisins, faisant augmenter ainsi la consommation globale d'énergie et le délai d'acheminement des données.

En effet, sur la figure 13 pour l'architecture conventionnelle « *Mesh-PDR* », on constate la présence de trois pics significatifs de consommation énergétique correspondant aux noeuds N3 et N11, qui sont des principaux noeuds relais pour les noeuds situés sur les membres mobiles et le bas du corps et le noeud N7 (principal noeud relais pour les noeuds situés à l'arrière du corps et au niveau de la tête).

En revanche, la nouvelle architecture « *Mesh-PDR + liens court terme* »permet un gain global de consommation d'énergie d'environ 30% par rapport à l'architecture classique « *Mesh-PDR* » tout en garantissant le même taux de livraison de données (voir figure 12).

Enfin, le nombre moyen de sauts par noeud vers le coordinateur N1 est illustré sur la Figure 14. Cette métrique reflète la longueur des chemins de routage et influe directement sur le délai d'acheminement des données obtenu.

On constate que l'utilisation du procédé selon l'invention permet une réduction globale d'environ 24% de la longueur des chemins de routage par rapport à l'architecture « Mesh-PDR », grâce à une exploitation astucieuse des liens fiables par intermittence. Ce gain peut atteindre 50% dans certains cas (e.g. lien N1-N8), minimisant ainsi significativement le délai d'acheminement des données.

On comprend donc que les procédés selon l'invention permettent d'améliorer les performances des réseaux corporels avec des dispositifs sans-fil en termes de connectivité, consommation d'énergie, latence, fiabilité, taux de livraison des données, fiabilité des communications, robustesse des protocoles face à la variation dynamique des conditions de propagation et/ou à la mobilité du corps humain, ceci sans engendrer de coûts important supplémentaires.

**Revendications**

1. Procédé d'évaluation de la qualité des liens radio pour un réseau corporel sans-fils comprenant au moins un premier et un second dispositifs sans-fil (N1, N2, N3, N4, N5, N6) formant un réseau corporel et susceptibles de communiquer entre eux, au moins l'un des deux dispositifs sans fil est susceptible d'être mobile par rapport à l'autre dans lequel

   - on exploite des messages reçus par au moins un des dispositifs sans-fil (N1, N2, N3, N4, N5, N6) et on mesure la qualité instantanée des liens radios ($l_{i,j}$) correspondant et on estime des durées pendant et entre lesquelles un lien radio est fiable, **caractérisé en ce que**
   - on calcule un indicateur de fiabilité des durées estimées et
   - on classifie les liens radio en fonction de l'indicateur de fiabilité en au moins deux catégories ($T_{i,j}$) dont une catégorie relative aux liens radio fiables par intermittence tenant compte de la mobilité des dispositifs sans fil du réseau corporel.

2. Procédé selon la revendication 1, **caractérisé en ce que** les messages échangés entre les dispositifs sans-fils sont des messages de service, des messages de données ou une combinaison de ces messages.

3. Procédé selon l'une quelconque des revendications 1 à 2, **caractérisé en ce que** l'on évalue la qualité instantanée des liens radio en mesurant au moins un indicateur radio tel que RSSI, LQI, ou SNR.

4. Procédé selon l'une quelconque des revendications 1 à 3, **caractérisé en ce que** l'on évalue la qualité instantanée des liens radio en mesurant un paramètre de connectivité.

5. Procédé selon la revendication 3 ou 4, **caractérisé en ce que** l'on détermine le paramètre de connectivité et / ou l'indicateur radio de façon binaire ayant une valeur « 1 » ou une valeur « 0 » $L_{i,j}^{[t]} = \{0,1\}$ pour tout instant $t$, entre un noeud $i$ et $j$, $i \neq j$.

6. Procédé selon la revendication 5, **caractérisé en ce que**

$$L_{i,j}^{[t]} = \begin{cases} 1 & \text{si le lien est actif par exemple si un échange de message a abouti} \\ 0 & \text{si le lien est inactif par exemple si un échange de message a échoué.} \end{cases}$$

7. Procédé selon la revendication 5 ou 6, **caractérisé en ce que**

$$L_{i,j}^{[t]} = \begin{cases} 1 & \text{si l'indicateur radio est supérieur ou égal à un seuil} \\ 0 & \text{si l'indicateur radio est inférieur à un seuil} \end{cases}$$

8. Procédé selon la revendication 6 ou 7, **caractérisé en ce que** pour classifier les liens radio selon au moins deux catégories, on détermine à partir du paramètre de connectivité ou à partir de l'indicateur radio pour respectivement deux dispositifs sans-fil (Ni, Nj), des durées de contact et d'inter-contact et on détermine la présence de motifs répétitifs de contact par intermittence.

9. Procédé selon la revendication 8, **caractérisé en ce que** la durée de contact, $\hat{C}_{i,j}^{[t]}$, est calculée comme étant la durée d'une séquence binaire $L_{N+M} = \left\{ L_{i,j}^{[1]}, L_{i,j}^{[2]}, ..., L_{i,j}^{[N+M]} \right\} \in \{1\}^N$ et $\{0\}^M$ et telle que $\dfrac{N-M}{N} \geq \gamma_F$ , où $\gamma_F$ est un seuil de fiabilité du lien radio.

10. Procédé selon la revendication 9, **caractérisé en ce que** la durée d'inter-contact, $\hat{I}_{i,j}^{[t]}$ , est calculée comme étant la durée d'une séquence binaire $L_{N+M} = \left\{ L_{i,j}^{[1]}, L_{i,j}^{[2]}, ..., L_{i,j}^{[N+M]} \right\} \in \{1\}^N$ et $\{0\}^M$ et telle que $N \leq \gamma_{UP}$, où $\gamma_{UP}$ est le nombre maximal de « 1 » toléré durant la période d'inter-contact.

11. Procédé selon la revendication 10, **caractérisé en ce que** l'on calcule les durées finales de contact et d'inter-contact selon les formules suivantes :

$$C_{i,j}^{[t]}(\alpha_{CT}) = \alpha_{CT} \times C_{i,j}^{[t-1]} + (1 - \alpha_{CT}) \times \hat{C}_{i,j}^{[t]}$$

$$I_{i,j}^{[t]}(\alpha_{CT}) = \alpha_{CT} \times I_{i,j}^{[t-1]} + (1 - \alpha_{CT}) \times \hat{I}_{i,j}^{[t]}$$

où

- $\hat{C}_{i,j}^{[t]}$ , respectivement $\hat{I}_{i,j}^{[t]}$ est l'estimation instantanée de la durée de contact, respectivement inter-contact ;

- $C_{i,j}^{[t]}$ , respectivement est l'estimation finale à l'instant $t$ de la durée de contact respectivement inter-contact; et
- $\alpha_{CT}$ est un facteur d'oubli.

12. Procédé selon la revendication 11, **caractérisé en ce que** l'on calcule un coefficient de variation glissant, $V_{i,j}^{[t]}$ , sur une fenêtre de taille $W_V$ qui est défini comme étant le rapport entre l'écart type et la moyenne des estimations sur une fenêtre glissante de taille $W_V$ et on le compare à un seuil $\gamma_V$ de sorte si le coefficient de variation glissant $V_{i,j}^{[t]}$ est inférieur au seuil $\gamma_V$, un lien est classifié comme fiable par intermittence et s'il est supérieur ou égal au seuil, sinon il est classifié comme étant non fiable.

13. Dispositif d'évaluation de la qualité des liens radio pour un réseau corporel sans-fils comprenant plusieurs dispositifs sans-fil comprenant au moins un premier et un second dispositifs sans-fil (N1, N2, N3, N4, N5, N6) formant un réseau corporel et susceptibles de communiquer entre eux, au moins l'un des deux dispositifs sans fil est susceptible d'être mobile par rapport à l'autre , pour la mise en oeuvre d'un procédé selon l'une quelconque des revendications 1 à 12, **caractérisé en ce qu'**il comprend des moyens configurés pour

- exploiter des messages reçus par au moins un des dispositifs sans-fil (N1, N2, N3, N4, N5, N6) et mesurer la qualité instantanée des liens radios ($I_{i,j}$) correspondant
- estimer des durées pendant et entre lesquelles un lien radio est fiable,
- calculer un indicateur de fiabilité des durées estimées et
- classifier les liens radio en fonction de l'indicateur de fiabilité en au moins deux catégories ($T_{i,j}$) dont une catégorie relative aux liens radio fiables par intermittence tenant compte de la mobilité des dispositifs sans fil du réseau corporel.

14. Procédé de transmission de messages à travers un réseau corporel sans-fil caractérisé en que l'on évalue la qualité des liens radio pour un réseau corporel sans-fils selon le procédé des revendications 1 à 12 pour le routage ou le relayage et en ce que pour la transmission des messages, on achemine les messages via un lien radio fiable par intermittence.

15. Procédé de transmission de messages selon la revendication 14, **caractérisé en ce que** l'on achemine les messages pendant une période pour laquelle le lien radio fiable par intermittence est considéré comme fiable.

16. Procédé selon la revendication 15, **caractérisé en ce que** l'on ordonnance des mesures de distance entre les dispositifs sans-fil en tenant compte de la prédiction d'apparition et/ou de disparition des liens fiables par intermittence.

17. Dispositif de transmission de messages à travers un réseau corporel sans-fil pour la mise en oeuvre d'un procédé selon l'une quelconque des revendications 14 à 15 caractérisé en qu'il comprend des moyens configurés pour évaluer la qualité des liens radio pour un réseau corporel sans-fils pour le routage d'échange de données et /ou relayage de paquets entre les dispositifs sans-fil selon le procédé des revendications 1 à 12 et des moyens configurés pour acheminer les messages via un lien radio fiable par intermittence pendant une période pendant laquelle le lien radio fiable par intermittence est considéré comme fiable.

**Patentansprüche**

1. Verfahren zur Bewertung der Qualität der Funkverbindungen für ein drahtloses Körpernetz, umfassend mindestens eine erste und eine zweite drahtlose Vorrichtung (N1, N2, N3, N4, N5, N6), die ein Körpernetz bilden und miteinander kommunizieren können, wobei mindestens eine der zwei drahtlosen Vorrichtungen in Bezug zur anderen beweglich sein kann, wobei

   - Meldungen ausgewertet werden, die von mindestens einer der drahtlosen Vorrichtungen (N1, N2, N3, N4, N5, N6) empfangen werden, und die momentane Qualität der entsprechenden Funkverbindung ($I_{ij}$) gemessen wird, und die Zeitdauern bewertet werden, während derer und zwischen denen eine Funkverbindung zuverlässig ist,

   **dadurch gekennzeichnet, dass**

   - ein Zuverlässigkeitsindikator der bewerteten Zeitdauern berechnet wird und
   - die Funkverbindungen in Abhängigkeit von dem Zuverlässigkeitsindikator in mindestens zwei Kategorien ($T_{ij}$) eingeteilt werden, wobei eine Kategorie bezüglich der zuverlässigen Funkverbindungen durch Intermittenz die Mobilität der drahtlosen Vorrichtungen des Körpernetzes berücksichtigt.

2. Verfahren nach Anspruch 1, **dadurch gekennzeichnet, dass** die zwischen den drahtlosen Vorrichtungen ausgetauschten Meldungen Dienstleistungsmeldungen, Datenmeldungen oder eine Kombination dieser Meldungen sind.

3. Verfahren nach einem der Ansprüche 1 bis 2, **dadurch gekennzeichnet, dass** die momentane Qualität der Funkverbindungen durch Messen mindestens eines Funkindikators, wie RSSI, LQI oder SNR, bewertet wird.

4. Verfahren nach einem der Ansprüche 1 bis 3, **dadurch gekennzeichnet, dass** die momentane Qualität der Funkverbindungen durch Messen eines Konnektivitätsparameters bewertet wird.

5. Verfahren nach Anspruch 3 oder 4, **dadurch gekennzeichnet, dass** der Konnektivitätsparameter und/oder der Funkindikator auf binäre Weise mit einem Wert "1" oder einem Wert "0" $L_{i,j}^{[t]} = \{0,1\}$ für jeden Zeitpunkt $t$ wischen einem Knoten $i$ und $j$ und $j$, $i \neq j$ bestimmt werden.

6. Verfahren nach Anspruch 5, **dadurch gekennzeichnet, dass**

$$L_{i,j}^{[t]} = \begin{cases} 1 & \text{wenn die Verbindung aktiv ist, beispielsweise wenn eine Meldung angekommen ist} \\ 0 & \text{wenn eine Verbindung inaktiv ist, beispielsweise wenn ein Meldungsaustausch gescheitert ist.} \end{cases}$$

7. Verfahren nach Anspruch 5 oder 6, **dadurch gekennzeichnet, dass**

$$L_{i,j}^{[t]} = \begin{cases} 1 & \text{wenn der Funkindikator größer oder gleich einer Schwelle ist} \\ 0 & \text{wenn der Funkindikator kleiner als eine Schwelle ist.} \end{cases}$$

8. Verfahren nach Anspruch 6 oder 7, **dadurch gekennzeichnet, dass** für die Einteilung der Funkverbindungen nach mindestens zwei Kategorien anhand des Konnektivitätsparameters oder anhand des Funkindikators für jeweils zwei drahtlose Vorrichtungen (Ni, Nj) Kontakt- und Interkontaktzeitdauerm bestimmt werden, und das Vorhandensein von sich wiederholenden Kontaktmotiven durch Intermittenz bestimmt wird.

9. Verfahren nach Anspruch 8, **dadurch gekennzeichnet, dass** die Kontaktzeitdauer $\hat{C}_{i,j}^{[t]}$ berechnet wird als die Dauer einer binären Sequenz $L_{N+M} = \left\{ L_{i,j}^{[1]}, L_{i,j}^{[2]}, ..., L_{i,j}^{[N+M]} \right\} \in \{1\}^N$ *und* $\{0\}^M$ und dass $\dfrac{N-M}{N} \geq \gamma_F$ ,

wobei $\gamma_F$ eine Zuverlässigkeitsschwelle der Funkverbindung ist.

10. Verfahren nach Anspruch 9, **dadurch gekennzeichnet, dass** die Interkontaktzeitdauer $\hat{I}_{i,j}^{[t]}$, berechnet wird als

die Dauer einer binären Sequenz $L_{N+M} = \left\{ L_{i,j}^{[1]}, L_{i,j}^{[2]}, ..., L_{i,j}^{[N+M]} \right\} \in \{1\}^N$ *und* $\{0\}^M$ und dass $N \leq \gamma_{UP}$, wobei $\gamma_{UP}$ die Maximalzahl "1" ist, die während des Interkontaktzeitraums toleriert wird.

11. Verfahren nach Anspruch 10, **dadurch gekennzeichnet, dass** die endgültigen Kontakt- und Interkontaktzeitdauern nach den folgenden Formeln berechnet werden:

$$C_{i,j}^{[t]}(\alpha_{CT}) = \alpha_{CT} \times C_{i,j}^{[t-1]} + (1 - \alpha_{CT}) \times \hat{C}_{i,j}^{[t]}$$

$$I_{i,j}^{[t]}(\alpha_{CT}) = \alpha_{CT} \times I_{i,j}^{[t-1]} + (1 - \alpha_{CT}) \times \hat{I}_{i,j}^{[t]}$$

wobei

- $\hat{C}_{i,j}^{[t]}$, bzw. $\hat{I}_{i,j}^{[t]}$ die momentane Bewertung der Kontakt- bzw. Interkontaktzeitdauer ist;

- $C_{i,j}^{[t]}$, bzw. $I_{i,j}^{[t]}$ die endgültige Bewertung zum Zeitpunkt $t$ der Kontakt- bzw. Interkontaktzeitdauer ist; und
- $\alpha_{CT}$ ein Unterlassungsfaktor ist.

12. Verfahren nach Anspruch 11, **dadurch gekennzeichnet, dass** ein gleitender Variationskoeffizient $V_{i,j}^{[t]}$ an einem Fenster der Größe $W_V$ berechnet wird, der als das Verhältnis zwischen der typischen Abweichung und dem Durchschnitt der Bewertungen an einem gleitenden Fenster der Größe $W_V$ definiert ist, und dieser mit einer Schweller $\gamma_V$ vergleichen wird, so dass, wenn der gleitende Variationskoeffizient $V_{i,j}^{[t]}$ kleiner als die Schwelle $\gamma_V$ ist, eine Verbindung als zuverlässig durch Intermittenz eingestuft wird, und wenn er größer oder gleich der Schwelle ist, sie anderenfalls als nicht zuverlässig eingestuft wird.

13. Vorrichtung zur Bewertung der Qualität der Funkverbindungen für ein drahtloses Körpernetz, umfassend mindestens mehrere drahtlose Vorrichtungen (N1, N2, N3, N4, N5, N6), die ein Körpernetz bilden und miteinander kommunizieren können, wobei mindestens eine der drahtlosen Vorrichtungen in Bezug zur anderen beweglich sein kann, für den Einsatz eines Verfahrens nach einem der Ansprüche 1 bis 12, **dadurch gekennzeichnet, dass** sie Mittel umfasst, die derart ausgeführt sind, dass

- Meldungen ausgewertet werden, die von mindestens einer der drahtlosen Vorrichtungen (N1, N2, N3, N4, N5, N6) empfangen werden, und die momentane Qualität der entsprechenden Funkverbindungen ($I_{ij}$) gemessen wird,
- die Zeitdauern, während derer und zwischen denen eine Funkverbindung zuverlässig ist, bewertet werden,
- ein Zuverlässigkeitsindikator der bewerteten Zeitdauern berechnet wird, und
- die Funkverbindungen in Abhängigkeit vom Zuverlässigkeitsindikator in mindestens zwei Kategorien ($T_{ij}$) eingeteilt werden, unter anderem eine Kategorie bezüglich der zuverlässigen Funkverbindungen durch Intermittenz, die die Mobilität der drahtlosen Vorrichtungen des Körpernetzes berücksichtigt.

14. Verfahren zur Übertragung von Meldungen durch ein drahtloses Körpernetz, **dadurch gekennzeichnet, dass** die Qualität der Funkverbindungen für ein drahtloses Körpernetz nach dem Verfahren der Ansprüche 1 bis 12 zum Weiterleiten oder Übertragen bewertet wird, und dass für die Übertragung der Meldungen die Meldungen über eine zuverlässige Funkverbindung durch Intermittenz befördert werden.

15. Verfahren zur Übertragung von Meldungen nach Anspruch 14, **dadurch gekennzeichnet, dass** die Meldungen während einer Zeitspanne, für die die zuverlässige Funkverbindung durch Intermittenz als zuverlässig betrachtet wird, befördert werden.

16. Verfahren nach Anspruch 15, **dadurch gekennzeichnet, dass** Distanzmessungen zwischen den drahtlosen Vorrichtungen unter Berücksichtigung der Vorhersage des Erscheinens und/oder Verschwindens der zuverlässigen Verbindungen durch Intermittenz verordnet werden.

17. Vorrichtung zur Übertragung von Meldungen durch ein drahtloses Körpernetz für den Einsatz eines Verfahrens nach einem der Ansprüche 14 bis 15, **dadurch gekennzeichnet, dass** sie Mittel umfasst, die derart ausgeführt sind, dass sie die Qualität der Funkverbindungen für ein drahtloses Körpernetz für das Weiterleiten eines Datenaustausches und/oder das Übertragen von Paketen zwischen drahtlosen Vorrichtungen nach dem Verfahren der Ansprüche 1 bis 12 bewerten, und Mittel, die derart ausgeführt sind, dass sie Meldungen über eine zuverlässige Funkverbindung durch Intermittenz während einer Zeitspanne, während der die zuverlässige Funkverbindung durch Intermittenz als zuverlässig betrachtet wird, befördern.

## Claims

1. A method for evaluating the quality of radio links for a wireless body area network comprising at least first and second wireless devices (N1, N2, N3, N4, N5, N6) forming a body area network and capable of communicating with one another, at least one of the two wireless devices having the capacity to be mobile relative to the other, whereby :

- Messages received by at least one of the wireless devices (N1, N2, N3, N4, N5, N6) are exploited to measure the instantaneous quality of the corresponding radio links ($I_{ij}$) and to estimate times during and between which a radio link is reliable, **characterised in that**:
- An estimated times reliability indicator is calculated; and
- The radio links are classified as a function of the reliability indicator into at least two categories ($T_{ij}$), one of which is a category relating to intermittently reliable radio links taking into account the mobility of the wireless devices of the body area network.

2. A method according to claim 1, **characterised in that** the messages exchanged between the wireless devices are service messages, data messages or a combination of these messages.

3. A method according to either of claims 1 to 2 **characterised in that** the instantaneous quality of the radio links is evaluated by measuring at least one radio indicator such as RSSI, LQI, or SNR.

4. A method according to any of claims 1 to 3 **characterised in that** the instantaneous quality of the radio links is evaluated by measuring a connectivity parameter.

5. A method according to claim 3 or 4 **characterised in that** the connectivity parameter and/or radio indicator is determined as a binary figure, having a value "1" or a value "0" $L_{i,j}^{[t]} = \{0,1\}$ for any instant $t$, between a node $i$ and $j$, $i \neq j$.

6. A method according to claim 5, **characterised in that**

$$L_{i,j}^{[t]} = \begin{cases} 1 & \text{if the link is active, for example if a message exchange is successful} \\ 0 & \text{if the link is inactive, for example if a message exchange has failed.} \end{cases}$$

7. A method according to claim 5 or 6, **characterised in that**

$$L_{i,j}^{[t]} = \begin{cases} 1 & \text{if the radio indicator is greater than or equal to a threshold} \\ 0 & \text{if the radio indicator is less than a threshold} \end{cases}$$

8. A method according to claim 6 or 7, **characterised in that** in order to classify the radio links into at least two categories, using the connectivity parameter or the radio indicator for two corresponding wireless devices (Ni, Nj), contact and inter-contact times are determined, as well as the presence of repetitive patterns of intermittent contact.

9. A method according to claim 8, **characterised in that** the contact time, $\hat{C}_{i,j}^{[t]}$, is calculated as being the duration of a binary sequence $L_{N+M} = \left\{ L_{i,j}^{[1]}, L_{i,j}^{[2]}, ..., L_{i,j}^{[N+M]} \right\} \in \{1\}^N$ et $\{0\}^M$ and such that $\frac{N-M}{N} \geq \gamma_F$ where $\gamma_F$ is a reliability threshold of the radio link.

10. A method according to claim 9, **characterised in that** the inter-contact time, $\hat{I}_{i,j}^{[t]}$, is calculated as being the duration of a binary sequence $L_{N+M} = \left\{ L_{i,j}^{[1]}, L_{i,j}^{[2]}, ..., L_{i,j}^{[N+M]} \right\} \in \{1\}^N$ et $\{0\}^M$ and such that $N \leq \gamma_{UP_1}$, where $\gamma_{UP}$ is the maximum number of "1" tolerated during the inter-contact period.

11. A method according to claim 10, **characterised in that** the final contact and inter-contact times are calculated by using the following formulae:

$$C_{i,j}^{[t]}(\alpha_{CT}) = \alpha_{CT} \times C_{i,j}^{[t-1]} + (1 - \alpha_{CT}) \times \hat{C}_{i,j}^{[t]}$$

$$I_{i,j}^{[t]}(\alpha_{CT}) = \alpha_{CT} \times I_{i,j}^{[t-1]} + (1 - \alpha_{CT}) \times \hat{I}_{i,j}^{[t]}$$

where

- $\hat{C}_{i,j}^{[t]}$, or respectively $\hat{I}_{i,j}^{[t]}$ is the instantaneous estimate of the contact time, or respectively inter-contact time;

- $C_{i,j}^{[t]}$, or respectively $I_{i,j}^{[t]}$ is the final estimate at the instant $t$ of the contact time, or respectively inter-contact time; and
- $\alpha_{CT}$ is a forget factor.

**12.** A method according to claim 11, **characterised in that** a sliding coefficient of variation, $V_{i,j}^{(n)}$, is calculated over a window of size $W_v$ that is defined as being a ratio between the standard deviation and the mean of the estimates over a sliding window of size $W_v$ and this is compared to a threshold [formula] so that if the sliding coefficient of variation, [formula] is less than the threshold [formula], a link is classified as intermittently reliable and if it is greater than or equal to the threshold, it is classified as unreliable.

**13.** An apparatus for evaluating the quality of radio links for a wireless body area network comprising several wireless devices comprising at least first and second wireless devices (N1, N2, N3, N4, N5, N6) forming a body area network and capable of communicating with each other, at least one of the two wireless devices having the capacity to be mobile relative to the other, for the implementation of a method according to any one of claims 1 to 12, **characterised in that** it includes means configured in order:

- To exploit messages received by at least one of the wireless devices (NI, N2, N3, N4, N5, N6) and to measure the instantaneous quality of the corresponding radio links (lij);
- To estimate the times during which a radio links is reliable;
- To calculate an estimated times reliability indicator; and
- To classify the radio links as a function of the said reliability indicator into at least two categories ($T_{ij}$) including a category relating to intermittently reliable radio links taking into account the mobility of the wireless devices of the body area network.

**14.** A method of transmitting messages across a wireless body area network **characterised in that** the quality of the radio links of a wireless body area network is evaluated using the method under claims 1 to 12 for routing via an intermittently reliable radio link.

**15.** A method of transmitting messages according to claim 14 **characterised in that** the messages are routed during a period for which the intermittently reliable radio link is deemed to be reliable.

**16.** A method according to claim 15, **characterised in that** measurements of distance between wireless devices is organised so as to take account of the predicted appearance and/or disappearance of intermittently reliable links.

**17.** An apparatus for transmitting messages across a wireless body area network for the implementation of a method according to any of claims 14 to 15 **characterised in that** it includes means configured in order to evaluate the quality of the radio links of a wireless body area network for data exchange routing and/or the relaying of packets between wireless devices according to the method in claims 1 to 12 and means configured in order to route messages via an intermittently reliable radio link during a period in which the intermittently reliable radio link is deemed to be reliable.

**FIG. 1**

**FIG. 2**

FIG. 3

Fréquence des mesures

Estimation périodique de la connectivité entre le noeud i et son voisinage
$\left\{ \Delta_{i,j}^{[t]}, \forall j \in n \wedge j \neq i \right\}$

10

Mise à jour historique connectivité
$\left\{ H_{i,j}^{[t]}(W), \forall j \in n \wedge j \neq i \right\}$

12B

Estimation qualité lien sur long terme
$\left\{ Q_{i,j}^{[t]}(W, \alpha_{LT}), \forall j \in n \wedge j \neq i \right\}$

Estimation qualité lien sur court terme
$\left\{ C_{i,j}^{[t]}(\alpha_{CT}), I_{i,j}^{[t]}(\alpha_{CT}), V_{i,j}^{[t]}(W_V), \forall j \in n \wedge j \neq i \right\}$

12C

12D

Classification des liens entre le noeud i et chacun de ses voisins

$T_{i,j}^{[t]} = \begin{cases} \text{fiable sur long terme} & \text{si} & Q_{i,j}^{[t]}(W, \alpha_{LT}) \geq \gamma_q \\ \text{fiable sur court terme} & \text{si} & \left\{ C_{i,j}^{[t]}, I_{i,j}^{[t]} \right\} > 0 \wedge V_{i,j}^{[t]}(C_{i,j}^{[t]}, I_{i,j}^{[t]}, W_V) < \gamma_V \\ \text{non fiable} & \text{sinon} \end{cases}, \forall j \in n \wedge j \neq i$

12E

Mise à jour de la table de voisinage du noeud i

| Noeud j | Caractéristiques lien (i-j) |
|---------|------------------------------|
| 1 | $\left\{ Q_{i,1}^{[t]}, T_{i,1}^{[t]}, C_{i,1}^{[t]}, I_{i,1}^{[t]}, V_{i,1}^{[t]}, \text{etc.} \right\}$ |
| ... | ... |
| n | $\left\{ Q_{i,n}^{[t]}, T_{i,n}^{[t]}, C_{i,n}^{[t]}, I_{i,n}^{[t]}, V_{i,n}^{[t]}, \text{etc.} \right\}$ |

12F

12

14

# FIG. 4

FIG. 5

FIG. 6

FIG. 7

FIG. 8

FIG. 9

FIG. 10

## FIG. 11

## FIG. 12

# FIG. 13

# FIG. 14

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010018517 A